# EUROPÄISCHE PATENTSCHRIFT

(11) **EP 2 822 406 B1**
(45) Veröffentlichungstag und Bekanntmachung des Hinweises auf die Patenterteilung: **30.11.2016**
(21) Anmeldenummer: 12712927.8
(22) Anmeldetag: 08.03.2012
(51) Int. Cl.: A23L 3/00, A23L 3/22

(54) **VERFAHREN, DOSIEREINRICHTUNG UND DOSIERVENTIL ZUR ASEPTISCHEN DOSIERUNG EINES FLÜSSIGEN ZUSATZSTOFFES IN EINE ERZWUNGENE STRÖMUNG EINES BASISPRODUKTS**
METHOD, METERING DEVICE AND METERING VALVE FOR THE ASEPTIC MEASURED DELIVERY OF A LIQUID ADDITIVE INTO A FORCED FLOW OF A BASE PRODUCT
PROCÉDÉ, DISPOSITIF DE DOSAGE ET SOUPAPE DE DOSAGE, POUR LE DOSAGE ASEPTIQUE D'UN ADDITIF LIQUIDE, DANS UN ÉCOULEMENT FORCÉ D'UN PRODUIT DE BASE

(43) Veröffentlichungstag der Anmeldung: 14.01.2015
(73) Patentinhaber: GEA TDS GmbH, 31157 Sarstedt (DE)
(72) Erfinder: ASSING, Hubert, 48683 Ahaus (DE); SCHMIED, Andreas, 48249 Dülmen (DE); SCHWENZOW, Uwe, 48683 Ahaus (DE); BUSS, Helmut, 46359 Heiden (DE); TASLER, Franz, 48653 Coesfeld (DE)
(74) Vertreter: Hauck Patentanwaltspartnerschaft mbB
(86) Internationale Anmeldenummer: PCT/EP2012/001020
(87) Internationale Veröffentlichungsnummer: WO 2013/131529

(56) Entgegenhaltungen:
- EP-A1- 0 447 760
- DE-A1- 10 337 057
- DE-A1-102007 056 833
- US-A1- 2002 172 745
- US-A1- 2005 031 751

## Beschreibung

### TECHNISCHES GEBIET

Die Erfindung betrifft ein Verfahren zur aseptischen Dosierung eines flüssigen Zusatzstoffes, insbesondere eines unter aseptischen Bedingungen erzeugten und bevorrateten Zusatzstoffes, in eine erzwungene Strömung eines Basisprodukts, das insbesondere gleichfalls unter aseptischen Bedingungen bereitgestellt wird, wobei der Zusatzstoff unter aseptischen Bedingungen mittels einer Entnahmeleitung aus einem Bevorratungsbehälter entnommen und über einen Endabschnitt der Entnahmeleitung dem Basisprodukt, das einen Produktraum durchströmt, zugeführt wird, sowie eine Dosiereinrichtung zur Durchführung des Verfahrens und ein Dosierventil für die Dosiereinrichtung.

### STAND DER TECHNIK

Flüssige aseptische Zusatzstoffe der vorgenannten Art, wie Enzyme, Aromen, Farben, Lipide, probiotische Bakterien und andere Nährstoffe werden in ein als aseptisches Basisprodukt der vorgenannten Art fungierendes Lebensmittel dosiert, um dieses mit besonderen Eigenschaften zu versehen. Da diese Zusatzstoffe hitzeempfindlich sein können, erfolgt die Zuführung dieser Additive in das Basisprodukt direkt vor dessen Abfüllung und nach dessen Wärmebehandlung zur Abtötung unerwünschter Keime, beispielsweise einer Ultra-Hoch-TemperaturBehandlung (UHT-Wärmebehandlung), und vorzugsweise im Rahmen einer sog. In-Line-Dosierung. Die Dosierung muss unter aseptischen Bedingungen und mit einem unter gleichfalls aseptischen Bedingungen bevorrateten sterilen Zusatzstoff erfolgen, damit das im Zuge der Wärmebehandlung aseptisch und damit keimfrei hergestellte Basisprodukt nicht in der Phase der Dosierung mit Keimen infiziert wird. DE10337057 offenbart ein Verfahren zur kaltaseptischen Abfüllung von Getränken und US 2002/172745 betrifft ein System zu in-line Beaufschlagung von Getränken.

Ein weitverbreiteter Anwendungsfall, bei dem ein Enzym als Zusatzstoff Verwendung findet, stellt die Herstellung lactosereduzierter oder lactosefreier Produkte dar. Hier wird Lactase zur enzymatischen Spaltung der Lactose eingesetzt. Konsumiert werden diese Produkte vor allem von Menschen, die an einem Mangel an ß-Galactosidase im Verdauungstrakt leiden. Eine weitere Einsatzmöglichkeit für lactosereduzierte Milch und verwandte Substanzen ist der Markt für kalorienreduzierte Produkte. Bei der Lactosespaltung entsteht je ein Molekül D-Glucose und β-D-Galactose [TÖPEL, 2004, S.99; [1] JEKLE; Lebensmitteltechnologisches Seminar, Lactosefreie und -reduzierte Milchprodukte, 2004]. Jedes dieser Moleküle hat eine größere Süßkraft als Lactose, so dass bei unverändertem Nährwert ein wesentlich süßeres Produkt hergestellt werden kann. Um eine nahezu vollständige Hydrolyse der Lactose zu gewährleisten, kann z.B. das Enzym β-Galactosidase bei der Produktion eingesetzt werden. Um die Prozesskosten der Hydrolyse reduzieren zu können, wurden Verfahren entwickelt, bei denen das Enzym unmittelbar vor der Abfüllung zugegeben wird **[1].** Man hat hierbei den Vorteil, dass die Distributionsphase als Hydrolysezeit genutzt werden kann und so geringere Enzymdosagen realisierbar sind. Die Dosierungsrate des in Rede stehenden Enzyms bewegt sich in einem Bereich von 0,2 bis 4 ml Enzym/Liter Basisprodukt (200 bis 4000 ppm).

In der Druckschrift der **Firma Tetra Pak Processing GmbH,** Tetra FlexDos™, Flexibles aseptisches Dosiersystem für flüssige Zusatzstoffe, **PD 10080 de 2007-02,** ist eine Dosiereinrichtung beschrieben, mit der unter Verwendung eines in Form eines flexiblen Beutels ausgebildeten Bevorratungsbehälters, in dem ein Zusatzstoff der in Rede stehenden Art bevorratet ist, eine aseptische In-Line-Dosierung dieses flüssigen Zusatzstoffes in ein Basisprodukt vorgenommen wird. Zu diesem Zweck wird der unter aseptischen Bedingungen mit dem sterilen Zusatzstoff gefüllte gebrauchsfertige 5- oder 10-Liter-Beutel, der seinerseits in einer eimerartigen Umverpackung Aufnahme findet, in die Dosiereinrichtung eingehängt. Im Zuge der Dosierung wird zur Überführung des Zusatzstoffes aus dem Beutel in das Basisprodukt eine als Entnahmeanordnung fungierende separate Schlauchanordnung verwendet, die vor der Dosierung über einen speziellen Adapter an den Beutel angeschlossen wird. Schlauchanordnung und Beutel werden getrennt voneinander her- und bereitgestellt, sie stammen im Regelfall von unterschiedlichen Herstellern und werden erst in der Dosiereinrichtung zusammengeführt und miteinander fest verbunden. An dem dem Adapter abgewandten Ende der Schlauchanordnung ist eine Injektionsnadel angeordnet, über die der Zusatzstoff an einem Injektionspunkt mittels einer auf die Schlauchanordnung von außen einwirkenden Schlauchradpumpe in das Basisprodukt eindosiert wird. Der Injektionspunkt befindet sich an einem Rohrstutzen einer Rohrleitung, in der das Basisprodukt strömt. Der Injektionspunkt ist dabei als Kammer ausgebildet, die dann als sterilisierende Dampfbarriere fungiert, wenn sie von geeignetem Sterildampf durchströmt wird. Zwei in Reihe geschaltete Membrane, die erste zwischen der Umgebung und dem Innenraum der Kammer, die zweite zwischen dem Innenraum der Kammer und dem Innenraum der Rohrleitung, müssen von der Injektionsnadel durchstochen werden, bevor die Dosierung beginnen kann.

Die bekannte Dosiereinrichtung mit dem zur Anwendung kommenden Beutel und der separaten Schlauchanordnung weisen insbesondere im Bereich des Beutels und der Ankopplung der Schlauchanordnung sowie am Injektionspunkt Nachteile auf und bedingen kritische Behandlungsschritte, die die absolut erforderliche Sicherheit der Sterilität des Dosierprozesses in Frage stellen können.

Der Entnahmeanschluss am Beutel kann vor der Ankopplung der Schlauchanordnung schädlichen Umgebungseinflüssen und/oder möglichen fehlerhaften Handlungen ausgesetzt sein, die die Sterilität der im Zuge des Kopplungsvorganges zusammengeführten, den zu dosierenden Zusatzstoff führenden kritischen Flächen beeinträchtigen können. Eine Sterilisierung dieser kritischen Flächen nach Vollzug des Kopplungsvorganges ist nicht mehr möglich. Da sich der Beutel und die Schlauchanordnung getrennt voneinander jeweils in einer Umverpackung befinden, ergibt sich allein durch diese Aufbewahrungs- und Handhabungssituation eine relativ kostspielige und wenig anwenderfreundliche Ausgangslage sowie eine nicht nur theoretische Möglichkeit zur Kontamination der kritischen Flächen mit Keimen.

Am Injektionspunkt muss darauf vertraut werden, dass während des Ein- und Durchstechvorganges der Injektionsnadel eine hinreichende Sterilisierung dieser stattfindet. Dabei ist weder die Verweilzeit der Injektionsnadel in der Dampfbarriere definiert oder konditioniert noch ist eine Temperaturkontrolle an der bewegten Injektionsnadel möglich. Eine Unterbrechung der Dosierung ist dann mit Blick auf den Zustand der innenseitigen Beaufschlagung der Injektionsnadel mit Zusatzstoff problematisch, wenn diese wegen einer Prozessunterbrechung aus ihrer Dosierposition temporär entfernt und, weil der Beutel mit hochwertigem Zusatzstoff noch teilweise befüllt ist und letzterer noch verbraucht werden soll, bei Fortsetzung der Dosierung erneut in ihre Dosierposition verbracht wird.

Sowohl bei der Ankopplung der Schlauchanordnung als auch bei der Sterilisierung der Injektionsnadel herrschen keine planmäßigen, reproduzierbaren und eindeutig konditionierten Bedingungen; zufällige und systematische Fehler können auftreten und die Sicherheit der aseptischen Dosierung in Frage stellen.

Es ist Zweck der vorliegenden Erfindung, ein Verfahren der gattungsgemäßen Art zu finden und eine Dosiereinrichtung zur Durchführung des Verfahrens sowie ein Dosierventil für die Dosiereinrichtung vorzuschlagen, die die Nachteile des Standes der Technik vermeiden und eine aseptische Dosierung sicherstellen, die durch planmäßige, reproduzierbare und eindeutig konditionierte und kontrollierte Bedingungen gekennzeichnet ist. Die vorliegende Erfindung hat sich insbesondere die Aufgabe gestellt, aseptische Kleinstmengen eines Zusatzstoffes bis maximal 250 ppm direkt in die erzwungene Strömung eines aseptischen Basisprodukts zu dosieren.

### ZUSAMMENFASSUNG DER ERFINDUNG

Der Zweck wird durch ein Verfahren mit den Merkmalen des Anspruchs 1 erfüllt. Vorteilhafte Ausgestaltungen des Verfahrens sind in den Unteransprüchen beschrieben. Eine Dosiereinrichtung zur Durchführung des Verfahrens ist Gegenstand des abhängigen Anspruchs 11. Vorteilhafte Ausführungsformen der Dosiereinrichtung sind in den Unteransprüchen angegeben. Ein Dosierventil für die vorgeschlagene Dosiereinrichtung ist Gegenstand des abhängigen Anspruchs 19. Vorteilhafte Ausführungsformen sind in den Unteransprüchen angegeben.

Der erfinderische Grundgedanke besteht darin, dass ein endseitiger Teil des Endabschnitts der Entnahmeleitung, in welcher Form er auch immer ausgestaltet ist, bevor er temporär in seine Dosierposition und -bereitschaft gelangt, unter planmäßigen, reproduzierbaren und eindeutig definierten und kontrollierten Bedingungen zumindest sterilisiert wird. Bei dem endseitigen Teil kann es sich um einen integralen Teil des Endabschnitts oder um einen separat vom Endabschnitt ausgeführten Teil handeln, wobei im letzten Falle dieser separate Teil mit einer steuerbaren Auslassöffnung, beispielsweise in Gestalt eines kleinen Dosierventils, ausgebildet sein kann. Dabei befindet sich der endseitige Teil des Endabschnitt noch außerhalb des Produktraums, in dem die Dosierung stattfindet, und es werden Bereiche des endseitigen Teils des Endabschnitts, die in seiner späteren Dosierposition an der Durchdringungsstelle innerhalb eines dort notwendigen Dichtungsmittels und, bezogen auf den Produktraum, darüber hinaus auf der vom Produktraum abgewandten Seite des Dichtungsmittels liegen, in die Sterilisierung einbezogen. Erst nach zumindest dieser Sterilisierung, die vorzugsweise temperaturüberwacht erfolgt, gelangt der endseitige Teil des Endabschnitts in seine Dosierposition, wobei noch keine Verbindung zum Produktraum hergestellt ist und diese Position als Warteposition bezeichnet wird. In der Warteposition ist der endseitige Teil des Endabschnitts an seiner Durchdringungsstelle mittels des zuvor zumindest sterilisierten Dichtungsmittels abgedichtet. Nunmehr wird unter diesen eindeutig definierten und kontrollierten Bedingungen eine Verbindung zum Produktraum hergestellt und mit der aseptischen Dosierung begonnen.

Die erfindungsgemäße verfahrenstechnische Lösung manifestiert sich durch die folgenden Behandlungsschritte für den Endabschnitt, bevor letzterer den Zusatzstoff dem Basisprodukt zuführt:
(a) der Endabschnitt wird von einer Seite ein Stück weit in einen anfänglichen Raum temporär eingeführt oder dort permanent positioniert und an der Durchdringungsstelle zu diesem anfänglichen Raum abgedichtet, wobei letzterer wahlweise zum Produktraum hin zu öffnen ist;
(b) ein in den anfänglichen Raum abgedichtet hineinragender endseitiger Teil des Endabschnitts wird zumindest sterilisiert, wobei alle Bereiche des anfänglichen Raumes gleichfalls in diese Behandlung einbezogen sind;
(c) der endseitige Teil des Endabschnitts wird in einen von dem anfänglichen Raum wahlweise dicht abteilbaren Teilraum um ein Stück hineinverschoben, wobei der endseitige Teil am Ende der Verschiebung an der Durchdringungsstelle zum Teilraum mittels eines nach Behandlungsschritt (b) behandelten Dichtungsmittels abgedichtet ist;
(d) der Teilraum wird zum Produktraum hin geöffnet.

Um zum Einen die Zeitspanne zwischen Sterilisierung und Dosierbeginn zu verkürzen und zum Anderen vor Allem ein Anbrennen des ggf. temperaturempfindlichen Zusatzstoffes im sterilisierten Bereich des Endabschnittes zu verhindern, ist es zweckmäßig, dass sich der Sterilisation nach Behandlungsschritt (b) eine Kühlung unter sterilen Bedingungen anschließt.

Die Sterilisation erfolgt in an sich bekannter Weise zweckmäßig mit sterilem Dampf (Sterildampf) und die Kühlung erfolgt zweckmäßig mit Sterilluft, wobei gemäß einem weiteren Vorschlag diese Temperaturbehandlung kontrolliert durchgeführt wird, indem die Sterilisation und die Kühlung jeweils temperaturüberwacht erfolgen. Erst wenn an der Temperaturmessstelle, die, in Strömungsrichtung des jeweiligen Fluids gesehen, unterstromig des zu behandelnden endseitigen Teils der Entnahmeleitung angeordnet ist, ein vorgegebener Temperaturgrenzwert erreicht ist, wird die jeweilige Temperaturbehandlung beendet. Dabei wird die Sterilisation in zielführender Weise bei einer Temperatur oberhalb von 125 °C durchgeführt und die Kühlung erfolgt auf eine Temperatur unterhalb 50 °C.

Um sicherzustellen, dass der Zusatzstoff nicht vorzeitig in den Endabschnitt gelangt, wird im Verlauf des vorgeschlagenen Verfahrens dafür gesorgt, dass die Entnahmeleitung bis zum Vollzug des Behandlungsschrittes (b) oder (c) frei bleibt vom Zusatzstoff und dass erst anschließend der Zusatzstoff bis zur Austrittsstelle des endseitigen Teils verbracht wird.

Da nicht auszuschließen ist, dass der Zusatzstoff beim Vorschieben zur Austrittsstelle des endseitigen Teils dort in mehr oder weniger geringen Mengen austritt und dadurch die Dosierung zumindest anfänglich verfälscht werden kann, sieht ein weiterer Vorschlag vor, dass wahlweise eine Spülung des anfänglichen Raumes mit sterilem Kondensat (Sterilkondensat) unterhalb einer Temperatur von 50 °C durchgeführt wird. Diese Spülung kann nach dem Behandlungsschritt (b) oder (c) durchgeführt werden, wobei nach dem Behandlungsschritt (c) zunächst der anfängliche Raum herzustellen ist, bevor die Spülung veranlasst wird. Um den anfänglichen Raum von dem dort nach der Spülung verbleibenden sterilen Kondensat zu befreien, ist es zweckmäßig, dass im Anschluss an die Spülung des anfänglichen Raumes dieser mittels Sterilluft ausgeblasen wird.

Durch messtechnische Vorkehrungen ist sichergestellt, dass der Zusatzstoff mengenproportional zum strömenden Basisprodukt dosiert wird. Die notwendige Dosiergenauigkeit wird erreicht, wenn der Transport des Zusatzstoffes in der Entnahmeleitung und ggf. auch die Entnahme aus dem Bevorratungsbehälter in an sich bekannter Weise zwangsweise erfolgen.

Die Dosiereinrichtung zur Durchführung des erfindungsgemäßen Verfahrens geht in an sich bekannter Weise aus von einer Dosiereinrichtung mit einem Produktleitungsabschnitt, der von dem Basisprodukt zwangsweise durchströmt ist und die mit wenigstens einem Bevorratungsbehälter für den Zusatzstoff ausgestattet ist, der jeweils über die Entnahmeleitung mit einer zugeordneten Dosierstelle verbunden ist. Im Regelfall sind zwei Bevorratungsbehälter vorgesehen, die im Wechsel betrieben werden, um so eine kontinuierliche Dosierung sicherzustellen. Weiterhin sind dritte Mittel zur zwangsweisen Förderung des Zusatzstoffes, erste und zweite Mitteln zur mengenmäßigen Erfassung des Basisprodukts und des Zusatzstoffes, vierte Mittel zur Bereitstellung von sterilem Dampf und eine der Dosiereinrichtung zugeordnete Steuereinrichtung vorgesehen. Die erfindungsgemäße Lösung ist dadurch gekennzeichnet, dass die dem jeweiligen Bevorratungsbehälter zugeordnete Dosierstelle jeweils innerhalb eines Dosierventils vorgesehen ist, das, jedes für sich, den Produktraum, in den der Produktleitungsabschnitt ein- und aus dem letzterer ausmündet, den anfänglichen Raum und den Teilraum sowie eine Aufnahme des Endabschnitts und des endseitigen Teils ausbildet.

Eine vorteilhafte Ausführungsform der Dosiereinrichtung sieht vor, dass jener Teil des anfänglichen Raumes, der nach Abteilung des Teilraumes verbleibt, einen Zulauf- und einen Ablaufkanal aufweist, die wahlweise jeweils über ein zugeordnetes Absperrventil absperrbar sind und wobei der Zulaufkanal wenigstens mit dem vierten Mittel zur Bereitstellung von sterilem Dampf verbindbar ist. Eine weitergehende Behandlung des anfänglichen Raumes im Rahmen einer Spülung, Kühlung oder eines Ausblasens wird auf einfache Weise dadurch möglich, dass der Zulaufkanal wahlweise mit einem fünften Mittel zur Bereitstellung von sterilem Kondensat und/oder einem sechsten Mittel zur Bereitstellung von Sterilluft verbindbar ist.

Eine Kontrolle, dass Sterilisation auf oder Kühlung unter eine hinreichende Temperatur erfolgt, wird zweckmäßig und einfach dadurch sichergestellt, dass im Ablaufkanal oder unterstromig unmittelbar im Anschluss an den Ablaufkanal ein Temperaturfühler angeordnet ist.

Höhere aseptische Sicherheit wird gegenüber dem bekannt gewordenen Stand der Technik dadurch erreicht, dass der Bevorratungsbehälter und wenigstens die Entnahmeleitung im Anlieferungszustand eine sterile Einheit bilden. Eine bevorzugte Ausführungsform des Bevorratungsbehälters sieht vor, diesen als flexiblen Beutel aus halbtransparentem Ethylenvenylazetat (EVA) herzustellen. Vor der Befüllung des Beutels mit dem Zusatzstoff ist die Entnahmeleitung mit dem Beutel fest und dicht verbunden. Diese Verbindung kann vorzugsweise form- und/oder kraftschlüssig verwirklicht sein, beispielsweise in Form einer elastisch duktilen Kupplung, sodass ein Fügen des Beutels mit der Entnahmeleitung zu einer fest und dicht verbundenen Einheit auf einfachste Weise gegeben ist. Die Verbindung kann aber auch stoffschlüssig ausgeführt werden, beispielsweise durch Verschweißung. In jedem Falle kann die Einheit, bestehend aus dem mit dem Zusatzstoff befüllten Beutel und der auf diesem vorzugsweise fixierten Entnahmeleitung in Verbindung mit der integrierten Injektionsnadel, dem festen Rohr mit glattem Ende oder dem Adapter, in einer PE-Umverpackung dicht eingeschlossen und in Gänze zum Zwecke der äußerlichen Sterilisierung einer geeigneten Behandlung, beispielsweise durch eine sterilisierende Bestrahlung, vorzugsweise einer Gammabestrahlung, ausgesetzt werden. Diese erfindungsgemäße Lösung beseitigt sicher die ansonsten bestehende Möglichkeit der Beeinträchtigung der Sterilität der Verbindung, wenn letztere erst nach der Befüllung des Beutels mit dem Zusatzstoff und vor der Dosierung des aseptischen Zusatzstoffes in das aseptische Basisprodukt hergestellt wird.

Um auch im Bereich eines Anschlusses des Beutels an die Entnahmeleitung nach der Befüllung des Beutels mit dem Zusatzstoff nachhaltig sterile Bedingungen herzustellen, sieht eine weitere Ausführungsform vor, dass in diesem Anschluss ein Knickventil angeordnet ist, mit dem durch irreversibles Brechen einer Barriere an einer Sollbruchstelle eine fluidgängige Verbindung zwischen dem Innenraum des Beutels und der Entnahmeleitung hergestellt wird. Die Verbindung mit der Entnahmeleitung wird erst kurz vor der Inbetriebnahme der Dosierung hergestellt, sodass die Entnahmeleitung bis zu diesem Zeitpunkt vollständig frei vom Zusatzstoff bleibt.

Damit eine derartige Anordnung nicht nur in der erfindungsgemäßen, sondern auch in bekannten Dosiereinrichtungen Verwendung finden kann, ist es zielführend, dass der Endabschnitt der Entnahmeleitung mit seinem integralen endseitigen Teil einstückig als Injektionsnadel ausgebildet ist. In anderen Fällen, in denen die Entnahmeleitung nicht über eine Injektionsnadel in die Dosierstelle einmündet, sondern nach Maßgabe des vorgeschlagenen Dosierverfahrens innerhalb des erfindungsgemäßen Dosierventils einen Anschluss findet, kann der Endabschnitt mit seinem integralen endseitigen Teil auch einstückig als Rohr mit glattem Ende, das fest ist oder duktil verformbar ist, ausgeführt sein.

Das Dosierverfahren und die Dosiereinrichtung zur Durchführung des Verfahrens ermöglichen aber auch die Verwendung eines Endabschnitts, bei dem der endseitige Teil separat vom Endabschnitt und mit einer steuerbaren Auslassöffnung, beispielsweise in Gestalt eines kleinen Dosierventils, ausgeführt ist. In diesem Falle kann der Endabschnitt wiederum als Injektionsnadel oder Rohr mit glattem Ende, das fest oder duktil verformbar ist, oder als Adapter zur Herstellung einer fluidgängigen Verbindung mit dem endseitigen Teil ausgebildet sein, wobei dann der Endabschnitt mit dem endseitigen Teil fluidgängig verbunden ist.

Ein erfindungsgemäßes Dosierventil für die Dosiereinrichtung ist u.a. durch folgende Baugruppen gekennzeichnet:
- einer ersten und einer zweiten Abdichteinrichtung für den endseitigen Teil der Entnahmeleitung;
- einer ersten Hülse, die den endseitigen Teil der Entnahmeleitung und die Entnahmeleitung aufnimmt und die durch einen Feder-Kolben-Antrieb mit einer zweiten Hubbewegung axial verschieblich angetrieben ist;
- einer Klemm- und Anpresseinrichtung, die mit dem Feder-Kolben-Antrieb axial verschieblich bewegt wird, die Entnahmeleitung axial und radial festlegt und die für den Anpressdruck in der ersten Abdichteinrichtung zur Erzeugung einer hinreichenden Dichtwirkung in einem ersten Dichtungsmittel an der Durchdringungsstelle des endseitigen Teils zum anfänglichen Raum sorgt.

Neben diesen Baugruppen weist das Dosierventil folgende Merkmale auf:
- ein in dem Produktleitungsabschnitt angeordnetes, den Produktraum berandendes Ventilgehäuse;
- einen das Ventilgehäuse einerseits verschließenden Gehäuseverschlusskörper mit einer Durchtrittsöffnung zum Produktraum;
- einen Stempel, der von der der Durchtrittsöffnung abgewandten Seite des Ventilgehäuses in dieses eingreift und nach einer ersten Hubbewegung in seiner einen Endlage, der Schließstellung, im Umfangsbereich der Durchtrittsöffnung, der als erste Sitzfläche ausgebildet ist, mit einer Stempelsitzfläche anliegt,
- eine in der der Durchtrittsöffnung zugewandten Stirnfläche des Stempels vorgesehene raumbildende Stempelausnehmung, die zusammen mit einem in dem Gehäuseverschlusskörper zwischen der Durchtrittsöffnung und dem stirnseitigen, verschieblichen Ende der ersten Hülse befindlichen Raum den anfänglichen Raum ausbildet, wobei der im Gehäuseverschlusskörper befindliche Raum über einen Zulauf- und einen Ablaufkanal jeweils mit der Umgebung des Dosierventils verbunden ist;
- einen Verschlusskolben, der von dem endseitigen Teil des Endabschnittes durchdrungen und auf einem an der ersten Hülse endseitig angeordneten, in den anfänglichen Raum hineinragenden Lagerzapfen angeordnet ist.

Besondere Bedeutung kommt im Rahmen der Erfindung dem Verschlusskolben in seinen durch die zweite Hubbewegung bestimmten Endlagen, einer Schließ- und einer Offenstellung, mit Blick auf die in ihm vorgesehene, auf ein zweites Dichtungsmittel einwirkende zweite Abdichteinrichtung zu:
- der Verschlusskolben liegt in seiner durch eine Antriebsfeder im Feder-KolbenAntrieb nach der zweiten Hubbewegung erzeugten Endlage, der Schließstellung, im Umfangsbereich der Durchtrittsöffnung, der als zweite Sitzfläche ausgebildet ist, dichtend an, wobei der Verschlusskolben in seiner Schließstellung zwischen sich und der Stempelausnehmung den Teilraum von dem anfänglichen Raum abteilt und der Verschlusskolben gegenüber dem endseitigen Teil durch das axial und radial verpresste zweite Dichtungsmittel abgedichtet ist;
- in einer mit der zweiten Hubbewegung erzeugten anderen Endlage des Verschlusskolbens, der Offenstellung, hat sich das zweite Dichtungsmittel spaltweit von dem endseitigen Teil gelöst und alle Oberflächen und Bereiche des anfänglichen Raumes sind der Beaufschlagung mit einem Fluid, dem sterilen Dampf, dem sterilen Kondensat oder der Sterilluft, zugänglich.

Die Konstruktion des Dosierventils wird vereinfacht, wenn der dem Ventilgehäuse abgewandte Endabschnitt des Gehäuseverschlusskörpers als Antriebskolben des Feder-Kolben-Antriebs ausgebildet und von der ersten Hülse verschieblich und abgedichtet durchdrungen ist, ein zugeordnetes Antriebsgehäuse topfartig ausgebildet und von der ersten Hülse durchdrungen und mit dieser form- und kraftschlüssig verbunden ist, und wenn sich die Antriebsfeder einerseits am Antriebskolben und andererseits an der ersten Hülse, vorzugsweise an einem an dieser ausgebildeten Federteller, in der Weise abstützt, dass die Rückstellkraft der Antriebsfeder den Verschlusskolben in seine Schließstellung überführt.

Eine weitere Ausführungsform sieht vor, dass das Antriebsgehäuse auf seiner dem Antriebskolben abgewandten Seite in ein topfförmiges, durch eine Überwurfmutter begrenztes Klemmgehäuse der Klemm- und Anpresseinrichtung übergeht, und dass die Überwurfmutter über konische Wirkflächen auf eine von dem Endabschnitt durchdrungene Zange dergestalt einwirkt, dass der Endabschnitt durch die Zange axial und radial festgelegt ist.

Eine hinreichende und dauerhafte Abdichtung des ersten Dichtungsmittels gegenüber dem endseitigen Teil wird dadurch sichergestellt, dass die Zange mit dem einen Ende einer in der ersten Hülse aufgenommenen, von dem Endabschnitt vollständig durchdrungenen zweiten Hülse verbunden ist und axial derart auf letztere einwirkt, dass diese mit ihrem anderen Ende das erste Dichtungsmittel axial und radial verpresst.

Eine hinreichende und dauerhafte Abdichtung des im Verschlusskolben angeordneten zweiten Dichtungsmittels gegenüber dem endseitigen Teil wird dadurch sichergestellt, dass der Verschlusskolben und der in diesen eingreifende Lagerzapfen einen den endseitigen Teil umschließenden keilförmigen Ringraum bilden, der sich von innen nach außen verjüngt und das zweite Dichtungsmittel aufnimmt. In der Schließstellung des Verschlusskolbens sind die Flanken des keilförmigen Ringraumes so weit angenähert, dass das zweite Dichtungsmittel axial und radial verpresst und damit gegen den endseitigen Teil abgedichtet ist. In der Offenstellung des Verschlusskolbens sind die Flanken des keilförmigen Ringraumes so weit voneinander entfernt, dass sich das zweite Dichtungsmittel aufweitet und spaltweit von dem endseitigen Teil entfernt ist.

### KURZBESCHREIBUNG DER ZEICHNUNGEN

Eine eingehendere Darstellung der Erfindung ergibt sich aus der folgenden Beschreibung und den beigefügten Figuren der Zeichnung sowie aus den Ansprüchen. Während die Erfindung in den verschiedensten Ausgestaltungen eines Verfahrens, einer Dosiereinrichtung und eines Dosierventils realisiert ist, werden in der Zeichnung ein Verfahren, eine Dosiereinrichtung und ein Dosierventil jeweils in einer bevorzugten Ausführungsform beschrieben. Es zeigen
- **Figur 1**: in sehr vereinfachter schematischer Darstellung eine Dosiereinrichtung zur Durchführung des erfindungsgemäßen Verfahrens, wobei lediglich eine einzige Dosiereinrichtung dargestellt ist, während im Regelfall mindestens zwei Dosiereinrichtungen im Wechsel betrieben werden;
- **Figur 2**: in perspektivischer Darstellung ein in seiner Meridianebene aufgeschnittenes Dosierventil für die Dosiereinrichtung gemäß **Figur 1****;** wobei die Meridianebene senkrecht zur Strömungsrichtung des in einem Produktleitungsabschnitt strömenden aseptischen Basisprodukts, in das ein aseptischer Zusatzstoff dosiert wird, orientiert ist und das Dosierventil sich in seiner Warteposition befindet;
- **Figur 3**: in einer Schnittdarstellung das in der Meridianebene gemäß **Figur 2** aufgeschnittene Dosierventil in einer dort mit **"B"** gekennzeichneten Blickrichtung;
- **Figur 4**: in einer Schnittdarstellung das Dosierventil gemäß **Figur 2** in einer weiteren Meridianebene, wobei der zugehörige Schnittverlauf in **Figur 3** mit **A-A** gekennzeichnet ist;
- **Figur 5**: die Schnittdarstellung des Dosierventils gemäß **Figur 3****,** wobei sich dieses nunmehr in seiner Sterilisationsposition befindet und
- **Figur 6**: die Schnittdarstellung des Dosierventils gemäß **Figur 3****,** wobei sich dieses nunmehr in seiner Dosierposition befindet.

### DETAILLIERTE BESCHREIBUNG

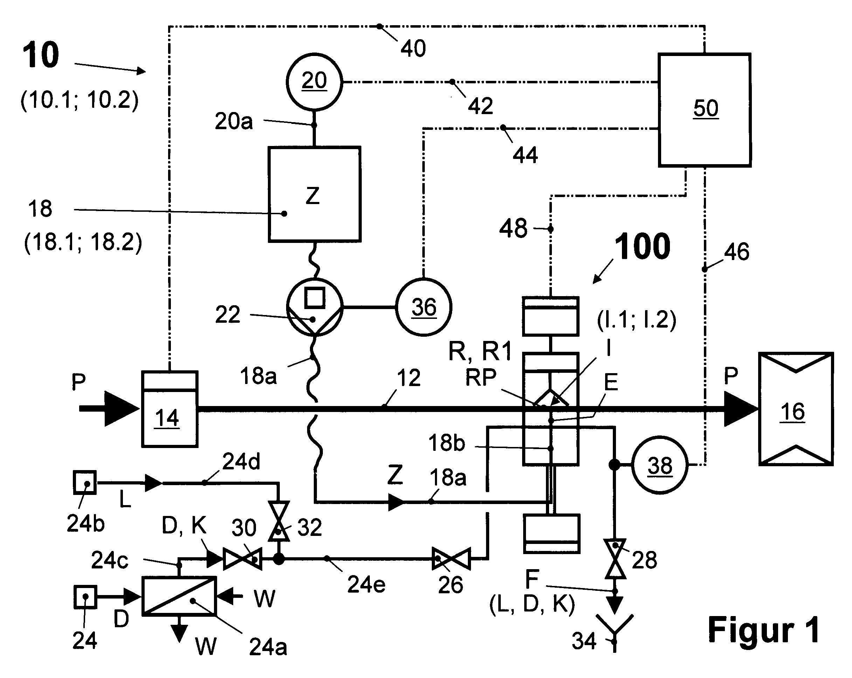

Eine Dosiereinrichtung 10 zur Durchführung des erfindungsgemäßen Verfahrens (**Figur 1**) ist im Regelfall wenigstens zweifach vorgesehen, wobei eine erste Dosiereinrichtung 10.1 und eine zweite Dosiereinrichtung 10.2 zur Sicherstellung eines kontinuierlichen Dosiervorganges, der über einen längeren Produktionszeitraum aufrechterhalten werden muss, nebeneinander in einem Produktionsleitungsabschnitt 12, der von einem aseptischen Basisprodukt P zwangsweise durchströmt ist und beispielsweise zu einer Füllmaschine 16 führt, angeordnet sind. Der Dosiereinrichtung 10 ist ein Bevorratungsbehälter 18 für einen aseptischen, flüssigen Zusatzstoff Z zugeordnet, der über eine Entnahmeleitung 18a mit einer an bzw. in dem Produktleitungsabschnitt 12 angeordneten Dosierstelle I verbunden ist. Bei zwei Dosiereinrichtungen 10.1, 10.2, die wechselweise betrieben werden, sind der ersten Dosiereinrichtung 10.1 ein erster Bevorratungsbehälter 18.1 und eine erste Dosierstelle 1.1 und der zweiten Dosiereinrichtung 10.2 ein zweiter Bevorratungsbehälter 18.2 und eine zweite Dosierstelle I.2 zugeordnet. Im Nachfolgenden beschränkt sich die Beschreibung auf den allgemeinen Aufbau einer Dosiereinrichtung 10, und es wird nicht mehr nach notwendigerweise dual vorhandenen Komponenten bei zwei nebeneinander angeordneten Dosiereinrichtungen 10.1, 10.2 differenziert.

In der Entnahmeleitung 18a ist ein drittes Mittel 22 zur zwangsweisen Förderung des Zusatzstoffes Z an die Dosierstelle I vorgesehen, beispielsweise eine rotierende Verdrängerpumpe, vorzugsweise eine Schlauchradpumpe. Bei einer Schlauchradpumpe wirkt der Fördermechanismus von außen auf die in Gestalt eines duktil verformbaren Schlauches ausgebildete Entnahmeleitung 18a in der Weise ein, dass keine unmittelbarer Kontakt zwischen dem Fördermechanismus und dem Zusatzstoff Z gegeben ist.

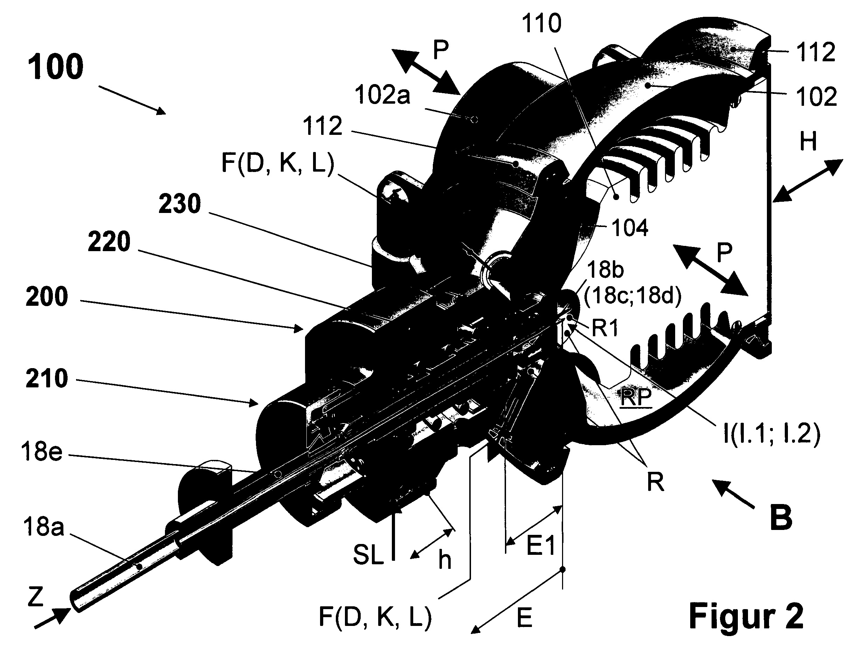

Eine weitere Möglichkeit, den Zusatzstoff Z zwangsweise an die Dosierstelle I zu fördern, besteht beispielsweise darin, den Zusatzstoff Z im Bevorratungsbehälter 18 unter Überdruck gegenüber dem Druck an der Austrittsstelle der Entnahmeleitung 18a an der Dosierstelle I zu setzen. Hierbei kann es sich um Druckbeaufschlagung des Bevorratungsbehälters 18 von innen, beispielsweise mit Sterilluft, oder auch um mechanische, hydraulische oder pneumatische Druckbeaufschlagung von außen handeln, wenn der Bevorratungsbehälter 18 in Form eines duktil verformbaren Beutels ausgebildet ist. Bei der erstgenannten Möglichkeit, der zwangsweisen Förderung beispielsweise durch eine Schlauchradpumpe, ist die Entnahmeleitung 18a an der Austrittsstelle eines endseitigen Teils E1 ihres Endabschnitts E (s. hierzu **Figur 2**) offen ausgeführt, und zwar in Form beispielsweise einer Injektionsnadel 18b oder eines festen oder duktil verformbaren Rohres mit glattem Ende 18c. Der endseitige Teil E1 ist hierbei integraler Teil des Endabschnitts E. Bei der zweitgenannten Möglichkeit, der zwangsweisen Förderung beispielsweise durch Druckbeaufschlagung des Bevorratungsbehälters 18, ist der endseitige Teil E1 separat vom Endabschnitt E und mit einer steuerbaren Auslassöffnung ausgeführt, wobei der Endabschnitt E wiederum als Injektionsnadel 18b oder festes oder duktil verformbares Rohr mit glattem Ende 18c oder als ein Adapter 18d zur Herstellung einer fluidgängigen Verbindung mit dem endseitigen Teil E1 ausgebildet sein kann und der Endabschnitt E mit dem endseitigen Teil E1 fluidgängig und unter aseptischen Bedingungen verbunden ist. Bei dieser Ausführungsform kann es sich bei dem endseitigen Teil E1 um ein kleines, ansteuerbares Dosierventil handeln, das permanent an der Dosierstelle I positioniert und wahlweise temporär mit den vorstehend genannten unterschiedlichen Ausgestaltungen 18b, 18c oder 18d des Endabschnitts E verbunden ist.

Die Dosiereinrichtung 10 ist mit einem oberstromig der Dosierstelle I in dem Produktleitungsabschnitt 12 angeordneten ersten Mittel 14, beispielweise einem Mengendurchflussmesser, zur Mengenerfassung des dort strömenden Basisprodukts P ausgestattet. Weiterhin ist ein zweites Mittel 20 vorgesehen, mit dem die Menge des der Dosierstelle I zuströmenden Zusatzstoffes Z erfasst wird. Bei dem zweiten Mittel 20 handelt es sich in einer bevorzugten Ausführungsform beispielsweise um eine hochpräzise Waage, an die über eine Aufhängung 20a der Bevorratungsbehälter 18 angehängt ist. Das dritte Mittel 22, die vorzugsweise verwendete Schlauchradpumpe, ist mit einer Drehzahlregeleinrichtung 36 verbunden. Erstes und zweites Mittel 14, 20 sowie das dritte Mittel 22 in Verbindung mit der Drehzahlregeleinrichtung 36 sind jeweils über Signalleitungen 40, 42 und 44 mit einer Steuereinrichtung 50 verbunden, sodass der Zusatzstoff Z mengenproportional zum strömenden Basisprodukt P dosiert werden kann.

Die Dosiereinrichtung 10 verfügt weiterhin über vierte Mittel 24 zur Bereitstellung von sterilem Dampf D (Sterildampf) für die Sterilisierung, dessen Temperatur den Sterilisationsbedingungen in an sich bekannter Weise anpassbar ist. Wird der sterile Dampf D über fünfte Mittel 24a, beispielsweise einen mit Weichwasser W beaufschlagten Wärmeaustauscher, geführt, dann kann durch diese Anordnung steriles Kondensat K (Sterilkondensat) für die Spülung im Rahmen des erfindungsgemäßen Verfahrens bereitgestellt werden. Darüber hinaus ist ein sechstes Mittel 24b zur Bereitstellung von steriler Luft L (Sterilluft) vorgesehen, mit der die Kühlung und das Ausblasen im Rahmen des erfindungsgemäßen Verfahrens bewerkstelligt werden kann. Steriler Dampf D und steriles Kondensat K werden über eine mittels eines dritten Absperrventils 30 schaltbare erste Leitung 24c und die Sterilluft L wird über eine mittels eines vierten Absperrventils 32 schaltbare zweite Leitung 24d einer dritten Leitung 24e zugeführt. Letztere ist über ein erstes Absperrventil 26 schaltbar und an ein Dosierventil 100 angeschlossen. Sie setzt sich, in Strömungsrichtung des jeweiligen Fluids F gesehen, das steriler Dampf D, steriles Kondensat K oder Sterilluft L sein kann, hinter dem Dosierventil 100 fort, kann dort über ein zweites Absperrventil 28 geschaltet werden und mündet in einen Gully 34. Die Temperatur des unterstromig vom Dosierventil 100 in der dritten Leitung 24e strömenden Fluids F kann über einen Temperaturfühler 38, der über eine vierte Signalleitung 46 mit der Steuereinrichtung 50 verbunden ist, gemessen werden. Die von der Steuereinrichtung 50 empfangenen Signale werden dort gemäß dem erfindungsgemäßen Verfahren verarbeitet, und es werden daraus Steuersignale für das Dosierventil 100 generiert, die letzterem über eine Steuerleitung 48 zugeführt werden.

Die Dosierstelle I ist innerhalb des Dosierventils 100 vorgesehen (**Figur 1**), wobei das Dosierventil 100 einen Produktraum RP, in den der Produktleitungsabschnitt 12 ein und aus dem letzterer ausmündet, einen anfänglichen Raum R und einen Teilraum R1 sowie eine Aufnahme des Endabschnitts E und des endseitigen Teils E1 ausbildet (**Figuren 2****,** **1**).

Jener Teil des anfänglichen Raumes R, der nach Abteilung des Teilraumes R1 verbleibt, weist einen Zulauf- und einen Ablaufkanal 104a, 104b auf (**Figur 2**), die wahlweise jeweils über das zugeordnete Absperrventil 26, 28 absperrbar sind und wobei der Zulaufkanal 104a wenigstens mit dem vierten Mittel 24 zur Bereitstellung von sterilem Dampf D verbindbar ist. Der Zulaufkanal 104a ist wahlweise mit dem fünften Mittel 24a zur Bereitstellung von sterilem Kondensat K und/oder dem sechsten Mittel 24b zur Bereitstellung von Sterilluft L verbindbar. Im Ablaufkanal 104b oder unterstromig unmittelbar im Anschluss an den Ablaufkanal 104b ist der Temperaturfühler 38 angeordnet.

Das Dosierventil 100 wird nachfolgend in den **Figuren 2** bis **6** beispielhaft in einer Ausführungsform beschrieben, bei der der Endabschnitt E der Entnahmeleitung 18a in Gestalt der Injektionsnadel 18b Aufnahme finden kann. Somit können der als duktiler Beutel ausgebildete Bevorratungsbehälter 18 für den Zusatzstoff Z in Verbindung mit der Injektionsnadel 18b, die im Stand der Technik seit längerer Zeit bekannt und eingeführt sind, in dem erfindungsgemäßen Dosierventil 100 uneingeschränkt zum Einsatz gebracht werden, ohne dass es mit Blick auf Beutel 18 und Injektionsnadel 18b an diesen irgendeiner Änderung oder Anpassung bedarf.

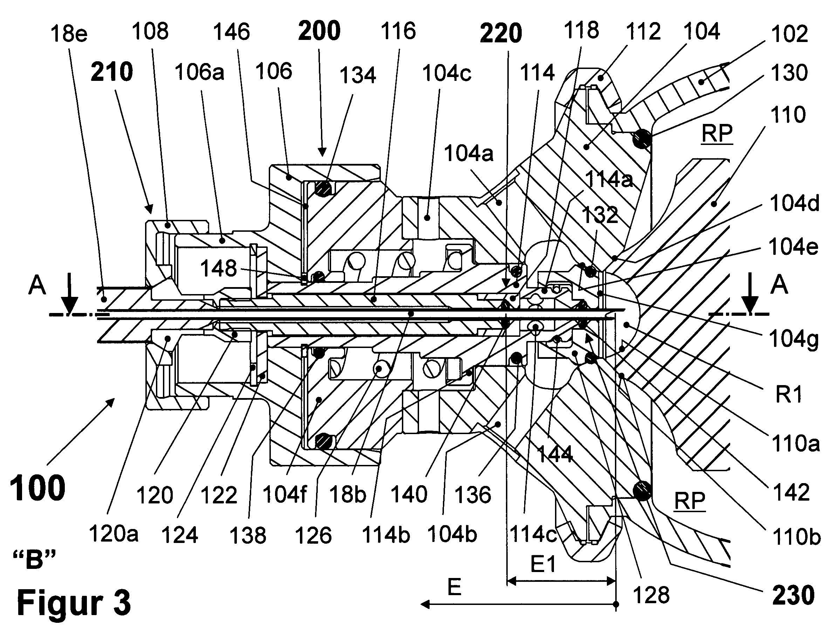

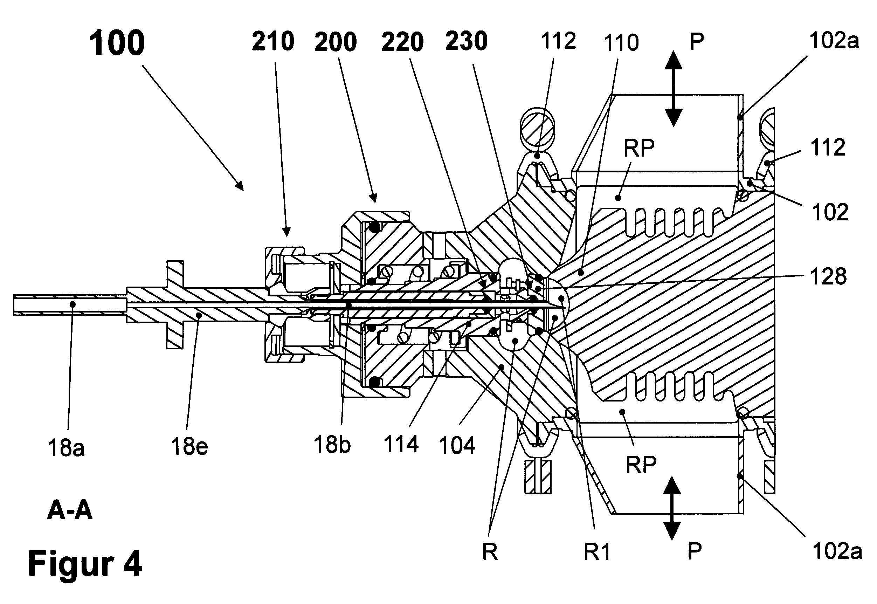

Das Dosierventil 100 weist den von einem Ventilgehäuse 102 berandeten Produktraum RP auf, wobei der Produktleitungsabschnitt 12 über zwei am Ventilgehäuse 102 angeformte Rohrstutzen 102a, die vorzugsweise einander gegenüberliegend angeordnet sind, in das Ventilgehäuse 102 ein- bzw. austritt (**Figuren 2****,** **4**). Vorzugsweise senkrecht zur Achse der Rohrstutzen 102a weist das Ventilgehäuse 102 eine nicht bezeichnete Öffnung auf, die von einem Gehäuseverschlusskörper 104 mittels einer Gehäusedichtung 130 dicht verschlossen ist. Der Gehäuseverschlusskörper 104 besitzt eine Durchtrittsöffnung 104g zum Produktraum RP (**Figur 3**). Eine erste Hülse 114, die koaxial zur Durchtrittsöffnung 104g angeordnet ist und verschieblich über eine erste und eine zweite Hülsendichtung 136, 138 abgedichtet von außen in den Gehäuseverschlusskörper 104 eingreift, ist mittels eines druckmittelbeaufschlagten Feder-Kolben-Antriebs 200 angetrieben und führt damit eine zweite Hubbewegung h aus. Dabei ist der dem Ventilgehäuse 102 abgewandte Endabschnitt des Gehäuseverschlusskörpers 104 als Antriebskolben 104f des Feder-Kolben-Antriebs 200 ausgebildet und von der ersten Hülse 114 verschieblich und abgedichtet durchdrungen. Ein zugeordnetes Antriebsgehäuse 106 ist topfartig ausgebildet, bildet mit dem eine zweite Kolbendichtung 134 außenseits tragenden Antriebskolben 104f einen mit Druckmittel, vorzugsweise Steuerluft SL (**Figur 2**), beaufschlagbaren Antriebsraum 146 und ist von der ersten Hülse 114 durchdrungen und mit dieser form- und kraftschlüssig verbunden. Eine Antriebsfeder 126 des Feder-Kolben-Antriebs 200 stützt sich einerseits am Antriebskolben 104f und andererseits an einem an der ersten Hülse 114 ausgebildeten Federteller 114b in der Weise ab, dass die Rückstellkraft der Antriebsfeder 126 die erste Hülse 114 in Richtung der Durchtrittsöffnung 104g verschiebt. Ein die Antriebsfeder 126 umgrenzender Raum ist über mindestens eine Laternenbohrung 104c mit der Umgebung des Dosierventils 100 verbunden.

Das Antriebsgehäuse 106 geht auf seiner dem Antriebskolben 104f abgewandten Seite in ein topfförmiges, durch eine Überwurfmutter 108 begrenztes Klemmgehäuse 106a einer Klemm- und Anpresseinrichtung 210 über, wobei die Überwurfmutter 108 über konische Wirkflächen auf eine von dem Endabschnitt E durchdrungene Zange 120a dergestalt einwirkt, dass der Endabschnitt E, der im vorliegenden Falle in diesem Bereich als ein Schaft 18e der Injektionsnadel 18b ausgebildet ist, durch die und gegenüber der Zange 120a axial und radial festgelegt ist. Die Zange 120a endet andererseits in einem Verbindungsteil 120, das mit einer in der ersten Hülse 114 angeordneten zweiten Hülse 116 verbunden ist.

Die erste Hülse 114 ist im Boden des Antriebsgehäuses 106 zwischen einer Scheibe 122, die am Boden einerseits anliegt und von einem im Klemmgehäuse 106a festgelegten ersten Sicherungsring 124 fixiert ist, und einem in der ersten Hülse 114 Aufnahme findenden dritten Sicherungsring 148 befestigt. Die zweite Hülse 116 stützt sich an ihrem dem Verbindungsteil 120 abgewandten Ende an einer ebenfalls in der ersten Hülse 114 angeordnete Andrückhülse 118 ab, die im Zusammenwirken miteinander eine erste Abdichteinrichtung 220 bilden. Die erste Abdichteinrichtung 220 ist vom Endabschnitt E vollständig durchdrungen und sie dichtet im Zusammenwirken mit der Klemm- und Anpresseinrichtung 210 den endseitigen Teil E1 des Endabschnittes E gegenüber der ersten Hülse 114 unmittelbar oder, wie im vorliegenden Fall, mittelbar durch ein axial und radial verpresstes erstes Dichtungsmittel 140 ab. Die zu dieser Verpressung notwendige axiale Kraft wird ursächlich von der Überwurfmutter 108 erzeugt und über die Zange 120a auf die zweite Hülse 116 übertragen.

Ein Stempel 110 greift von der der Durchtrittsöffnung 104g abgewandten Seite des Ventilgehäuses 102 in dieses ein und liegt nach einer ersten Hubbewegung H in seiner einen Endlage, der Schließstellung, im Umfangsbereich der Durchtrittsöffnung 104g, der als erste Sitzfläche 104d ausgebildet ist, mit einer Stempelsitzfläche 110b an. Eine Betätigungsstange des Stempels 110 und ihre Durchdringungsstelle durch das Ventilgehäuse 102 sind nicht dargestellt. Der Stempel 110 und die Durchdringungsstelle sind durch einen nicht bezeichneten Balg, vorzugsweise ein Faltenbalg oder Wellrohr, stoffschlüssig überbrückt. Das Ventilgehäuse 102 ist einerseits mit dem Gehäuseverschlusskörper 104 und andererseits mit einem nicht dargestellten Verschlussteil, das die Durchdringungsstelle und die Einspannung des Balges ausbildet, jeweils über eine Klemmringverbindung 112 verbunden.

In der der Durchtrittsöffnung 104g zugewandten Stirnfläche des Stempels 110 ist eine raumbildende Stempelausnehmung 110a vorgesehen (Figur 3), die zusammen mit einem in dem Gehäuseverschlusskörper 104 zwischen der Durchtrittsöffnung 104g und dem stirnseitigen, verschieblichen Ende der ersten Hülse 114 befindlichen Raum den anfänglichen Raum R ausbildet, wobei der im Gehäuseverschlusskörper 104 befindliche Raum über den Zulauf- und den Ablaufkanal 104a, 104b für das Fluid F, das steriler Dampf D, steriles Kondensat K oder Sterilluft L sein kann, jeweils mit der Umgebung des Dosierventils 100 verbunden ist (Figur 2).

Das Dosierventil 100 weist weiterhin einen Verschlusskolben 128 auf, der von dem endseitigen Teil E1 des Endabschnittes E durchdrungen und auf einem an der ersten Hülse 114 endseitig angeordneten, in den anfänglichen Raum R hineinragenden Lagerzapfen 114a, vorzugsweise axial und radial geringfügig begrenzt verschieblich, angeordnet ist. Der Verschlusskolben 128 liegt in seiner durch die Rückstellkraft der Antriebsfeder 126 nach der zweiten Hubbewegung h erzeugten Endlage, seiner Schließstellung, im Umfangsbereich der Durchtrittsöffnung 104g, der als zweite Sitzfläche 104e ausgebildet ist, vorzugsweise mit einer ersten Kolbendichtung 132 dichtend an. In dieser Schließstellung teilt der Verschlusskolben 128 zwischen sich und der Stempelausnehmung 110a den Teilraum R1 von dem anfänglichen Raum R ab und der Verschlusskolben 128 ist gegenüber dem endseitigen Teil E1 durch ein axial und radial verpresstes zweites Dichtungsmittel 142 im Rahmen einer zweiten Abdichteinrichtung 230 abgedichtet. In einer durch eine mit der zweiten Hubbewegung h erzeugten anderen Endlage des Verschlusskolbens 128, seiner Offenstellung, hat sich das zweite Dichtungsmittel 142 durch die ihm innewohnende Eigenelastizität spaltweit von dem endseitigen Teil E1 gelöst, und alle Oberflächen und Bereiche des anfänglichen Raumes R sind der Beaufschlagung mit dem Fluid F zugänglich.

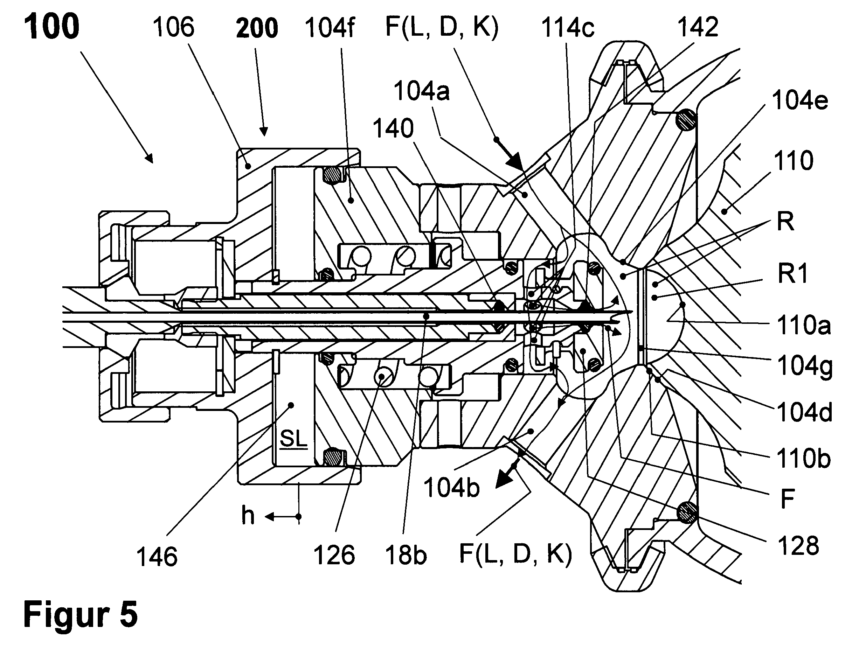

Die Verpressung und die Aufweitung des zweiten Dichtungsmittels 142 werden vorzugsweise dadurch erreicht, dass der Verschlusskolben 128 und der in diesen eingreifende Lagerzapfen 114a einen den endseitigen Teil E1 umschließenden keilförmigen Ringraum bilden, der sich von innen nach außen verjüngt und das zweite Dichtungsmittel 142 aufnimmt. In der Schließstellung des Verschlusskolbens 128 sind die Flanken des keilförmigen Ringraumes so weit angenähert, dass das zweite Dichtungsmittel 142 axial und radial verpresst und damit gegen den endseitigen Teil E1 abgedichtet ist. In der Offenstellung des Verschlusskolbens 128 sind die Flanken des keilförmigen Ringraumes so weit voneinander entfernt, dass sich das zweite Dichtungsmittel 142 unter der Wirkung seiner Eigenelastizität aufweitet und spaltweit von dem endseitigen Teil E1 entfernt ist. In diesem aufgeweiteten Zustand des zweiten Dichtungsmittels 142 ist dieses allseits mit Fluid F umspülbar, weil der Lagerzapfen 114a vorzugsweise einerseits bis zum zweiten Dichtungsmittel 142 hin innen hohl ausgebildet ist und andererseits dieser Hohlraum über wenigstens einen Kanal 114c mit der Umgebung des Lagerzapfens 114 und des Verschlusskolbens 128, dem anfänglichen Raum R, fluidgängig verbunden ist. Der Verschlusskolben 128 ist auf den Lagerzapfen 114a vorzugsweise mit geringem radialen Spiel aufgeschoben und dort über einen zweiten Sicherungsring 144 axial festgelegt. Diese Lagerung ist über geeignete, nicht bezeichnete Durchlässe im Schaft des Verschlusskolbens 128 gleichfalls fluiddurchgängig ausgestaltet (siehe hierzu auch **Figur 5****).**

Der Aufbau des Dosierventils 100 wurde vorstehend anhand der **Figuren 2** bis **4****,** in denen es sich in der sog. Warteposition befindet, und der **Figur 5****,** in der es sich in der Sterilisationsposition befindet, beschrieben. Die Einbettung des Dosierventils 100 in die Dosiereinrichtung 10 zeigt **Figur 1****.** In der Warteposition gemäß den **Figuren 2** bis **4** ist der Antriebsraum 146 nicht mit Druckmittel, der Steuerluft SL **(****Figur 2****),** beaufschlagt. Der Verschlusskolben 128 wird unter dem Einfluss der Rückstellkraft der Antriebsfeder 126 in seine abdichtende Schließlage auf die zweite Sitzfläche 104e gepresst. Das erste und das zweite Dichtungsmittel 140, 142 dichten die Injektionsnadel 18b in der vorstehend beschriebenen Weise ab. Vom anfänglichen Raum R **(****Figur 2****)** ist mittels des Verschlusskolbens 128 der im Wesentlichen von der raumbildenden Stempelausnehmung 110b gebildete Teilraum R1 abgeteilt (s. auch **Figuren 3** und **4****).** Zwischen dem Teilraum R1 und dem von dem Ventilgehäuse 102 berandeten Produktraum RP besteht noch keine fluidgängige Verbindung, weil sich der Stempel 110 mit seiner Stempelsitzfläche 110b noch in seiner Schließlage auf der zugeordneten ersten Sitzfläche 104d befindet **(****Figur 3****).**

In der Sterilisationsposition gemäß **Figur 5** ist der Verschlusskolben 128 durch Beaufschlagung des Antriebsraumes 146 mit Steuerluft SL in seine Offenstellung verschoben. Das erste Dichtungsmittel 140 bleibt verpresst und das zweite Dichtungsmittel 142 ist radial aufgeweitet, sodass der endseitige Teil E1 am ersten Dichtungsmittel 140 permanent positioniert und abgedichtet bleibt und von da aus von jedwedem Fluid F frei umspülbar in den anfänglichen Raum R eingreift. Über den Zulaufkanal 104a kann Fluid F, steriler Dampf D zur Sterilisation, Sterilluft L zur Kühlung, steriles Kondensat K zur Spülung oder Sterilluft L zum Ausblasen, zugeführt werden.

In der Offenstellung des Verschlusskolbens 128 ist, wenn der Stempel 110 um die erste Hubbewegung H gleichfalls in seine Offenstellung überführt ist, nach Durchführung einer aseptischen Dosierung, wenn der Produktleitungsabschnitt 12 üblicherweise planmäßig einer chemischen Reinigung, einer sog. CIP-Reinigung (cleaning in place), mittels Säure und/oder Lauge und Spülwasser unterzogen wird, auch eine diesbezügliche CIP-Reinigung aller relevanten Bereiche des Dosierventils 100 durchführbar, wobei das jeweilige Reinigungsmittel über den Produktleitungsabschnitt 12 zu- und über den Ablaufkanal 104b jeweils abgeführt wird.

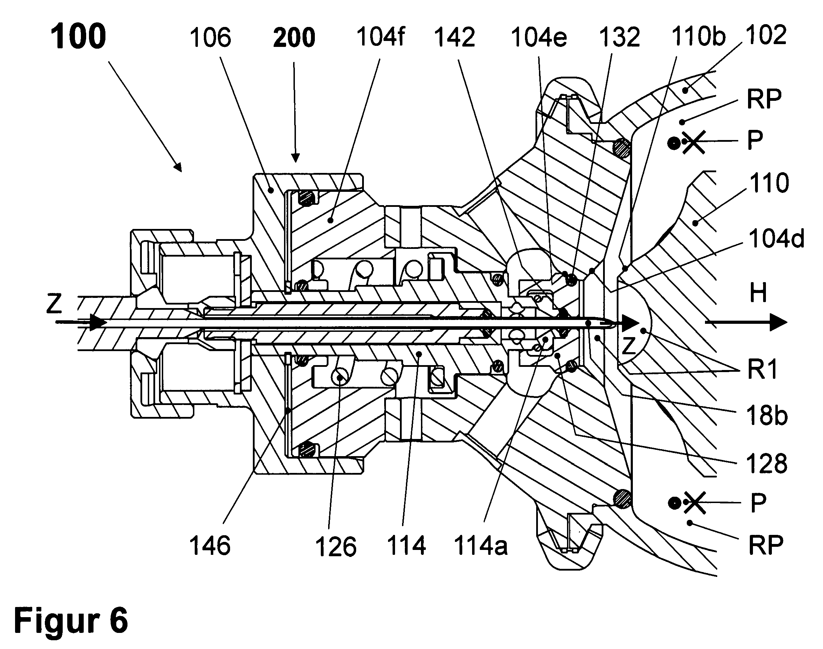

Nach der Sterilisation, der Kühlung und ggf. der Spülung mit der anschließenden Ausblasung gemäß **Figur 5** wird der Verschlusskolben 128 nach Wegfall der Steuerluft SL unter der Rückstellkraft der Antriebsfeder 126 zunächst wieder in seine Schließstellung überführt, die Warteposition gemäß den **Figuren 2** bis **4****.** Alsdann können der Stempels 110 um die erste Hubbewegung H in seine Offenstellung überführt und der Teilraum R1 zum Produktraum RP hin geöffnet werden, in dem das Basisprodukt P strömt. Das Dosierventil 100 befindet sich nunmehr in der Dosierposition gemäß **Figur 6****,** in der der Zusatzstoff Z über die Auslassöffnung des endseitigen Teils E1 des Endabschnitts E, im vorliegenden Falle der Injektionsnadel 18b, mengenproportional zum strömenden Basisprodukt P dosiert wird.

Anstelle der Injektionsnadel 18b kann auch in sinngemäß in gleicher Weise ein festes oder ein duktil verformbares Rohr mit glattem Ende 18c Verwendung finden. Der endseitige Teil E1 **(****Figur 3****)** des Endabschnitts E kann auch separat von letzterem und mit einer steuerbaren Auslassöffnung ausgeführt sein. Dies wäre dann beispielsweise ein steuerbares kleines Dosierventil. Die wahlweise Ankopplung dieses kleinen Dosierventils an den Endabschnitt E kann dann beispielsweise wiederum über eine Injektionsnadel 18b, ein festes oder duktil verformbares Rohr mit glattem Ende 18c oder einen Adapter 18d zur Herstellung einer fluidgängigen Verbindung erfolgen, wobei auf diese Gegebenheiten die verbleibenden erfindungsgemäßen verfahrenstechnischen Lösungsmerkmale sinngemäß zu übertragen sind.

Um die Sicherheit, die durch das vorgeschlagene Verfahren zur aseptischen Dosierung im Bereich des Dosierventils 100 erreicht wird, auch im Bereich eines als Beutel ausgebildeten Bevorratungsbehälters 18 und seiner Verbindung mit der Entnahmeleitung 18a in adäquater Weise zu erreichen, wird vorgeschlagen, dass der Beutel 18 und wenigstens die Entnahmeleitung 18a im Anlieferungszustand eine sterile Einheit bilden und in dieser Ausgestaltung in der Dosiereinrichtung 10 Verwendung finden **(****Figur 1****).** Die wesentlichen Merkmale dieser Einheit, ihre Vor- und Nachbehandlung, seien nachfolgend kurz beschrieben. Der Beutel 18, der vorzugsweise ein Fassungsvermögen von beispielsweise 5 oder 10 Liter besitzt, zur sterile Bevorratung von flüssigen aseptischen Zusatzstoffen Z, wie beispielsweise Enzyme, Aromen, Farben, Lipiden, probiotischen Bakterien und anderen Nährstoffen, ist in seinem unbefüllten Zustand ein flächiges, rechteckförmiges, aus einem geeigneten Kunststoff bestehendes Gebilde, wie es in seiner grundsätzlichen Form in der Medizintechnik Anwendung findet. Als Ausgangsform wird in der Regel eine Schlauchfolie mit geeignetem Durchmesser verwendet, die auf eine erforderliche axiale Länge konfektioniert und an den beiden offenen Enden dann durch Verschweißung geschlossen wird, wobei an oder in diesen Enden dann die jeweiligen anwendungsspezifischen Vorkehrungen, Besonderheiten oder Erfordernisse verwirklicht werden. Er besteht vorzugsweise aus halbtransparentem Ethylenvenylazetat (EVA) mit Lichtschutz.

In einem Anschluss zur mindestens die Entnahmeleitung 18a beinhaltenden Entnahmeanordnung, der vorzugsweise als Schlauchstutzen ausgeführt ist, ist, von außen am Schlauchstutzen zugänglich, ein sog. Knickventil angeordnet, mit dem durch irreversibles Brechen einer Barriere an einer Sollbruchstelle eine fluidgängige Verbindung zwischen dem Innenraum des Beutels 18 und der Entnahmeanordnung hergestellt wird. Die Entnahmeanordnung besteht aus der vorstehend bereits beschriebenen Entnahmeleitung 18a, vorzugsweise einem Entnahmeschlauch, die über eine Kupplung fest und dicht mit dem vorg. Anschluss verbunden ist. Hierbei kann es sich um eine form- und/oder kraftschlüssige oder auch um eine stoffschlüssige Verbindung handeln. Am der Kupplung abgewandten Ende der Entnahmeleitung 18a mündet diese in die beschriebene Injektionsnadel 18b, mit der der Zusatzstoff Z unter aseptischen Bedingungen in das in dem Produktleitungsabschnitt 12 strömende Basisprodukt P eindosiert wird. In Fließrichtung des Zusatzstoffes Z gesehen weist die Entnahmeanordnung vor der Injektionsnadel 18b vorzugsweise ein Rückschlagventil und davor einen Filter auf. Die Barriere des Knickventils wird erst nach Einhängung des Beutels 18 über eine bevorzugte Dreipunktaufhängung in die Dosiereinrichtung 10 und kurz vor der Inbetriebnahme der aseptischen Dosierung an der Sollbruchstelle gebrochen. Der Beutel 18 und die mit diesem fest verbundene und auf diesem fixierte Entnahmeanordnung werden als zusammengehörende Einheit mit einem geeigneten Mittel sterilisiert. Dies geschieht bereits vor der Befüllung des Beutels 18 mit dem Zusatzstoff Z.

Das vorstehend erwähnte Knickventil besitzt eine Einschnürstelle und diese fungiert als Sollbruchstelle. Ein hinreichendes Biegemoment quer zur Längsachse des Knickventils führt zu einem vollständigen Bruch des Knickventils an dieser Stelle, sodass das Knickventil in zwei Teile vereinzelt ist. Das Knickventil wird, mit dem freien Ende des ersten Teils zuerst, in den Anschluss eingeführt und dort so weit axial verschoben, dass nach Brechen des Knickventils an der Sollbruchstelle der vereinzelte, durchmesserkleinere erste Teil in den Beutel 18 gelangt, sodass ein unbehinderter Zugang vom Innenraum des Beutels 18 über einen durch den Bruch im zweiten Teil erst freigelegten Innendurchgang zur Entnahmeanordnung sichergestellt ist.

Ein Verfahren zur Befüllung des Beutels 18 mit dem flüssigen Zusatzstoff Z, zur Bevorratung des Zusatzstoffes Z in dem Beutel 18 und zur Entnahme des Zusatzstoffes Z aus dem Beutel 18, jeweils unter aseptischen Bedingungen und durchgeführt mit einem Beutel 18, wie er vorstehend beschrieben wurde, wird in den nachfolgend angegebenen Schritten durgeführt:
- Ein Umverpackungsbeutel wird durch Verschweißung seiner noch offenen Seite um den Beutel 18 herum vollständig hermetisch abgeschlossen.
- Der umverpackte Beutel 18 wird an die Dosiereinrichtung 10 verbracht, dort aus dem Umverpackungsbeutel entnommen und in der Dosiereinrichtung 10 über die Dreipunktaufhängung in seiner Aufhängeposition positioniert **(****Figur 1****).** Zum Schutz vor Beschädigungen beim Transport kann der Umverpackungsbeutel wiederum in einer Transportverpackung, beispielsweise in einem Karton, Aufnahme finden.
- Die Injektionsnadel 18b am Ende der Entnahmeanordnung findet im Dosierventil 100 entsprechend den **Figuren 2** bis **6** und der zugeordneten vorstehenden Beschreibung Aufnahme.
- Zeitlich gesehen wird kurz vor der Dosierung des flüssigen Zusatzstoffes Z in das in dem Produktleitungsabschnitt 12 strömende Basisprodukt P eine fluidgängige Verbindung zwischen dem Innenraum des Beutels 18 und der Entnahmeanordnung durch Brechen der Barriere an der Sollbruchstelle in dem Knickventil hergestellt.
- Der flüssige Zusatzstoff Z wird von dem Beutel 18 zur Austrittsstelle der Injektionsnadel 18b durch von außen auf die Entnahmeleitung 18b einwirkende dritte Mittel 22 zwangsweise und mengenproportional zum strömenden Basisprodukt P, gesteuert durch die Steuereinrichtung 50, gefördert.

### BEZUGSZEICHENLISTE DER VERWENDETEN ABKÜRZUNGEN

- 10: Dosiereinrichtung
- 10.1: erste Dosiereinrichtung
- 10.2: zweite Dosiereinrichtung

- 12: Produktleitungsabschnitt (für Basisprodukt P)
- 14: erstes Mittel (zur Mengenerfassung des Basisprodukts P)
- 16: Füllmaschine (oder Prozessaggregat, allgemein)

- 18: Bevorratungsbehälter (z.B. flexibler oder starrer Behälter)
- 18.1: erster Bevorratungsbehälter
- 18.2: zweiter Bevorratungsbehälter

- 18a: Entnahmeleitung (z.B. Schlauch; feste oder duktile Rohrleitung)
- 18b: Injektionsnadel (Endabschnitt E)
- 18c: Rohr mit glattem Ende (Endabschnitt E)
- 18d: Adapter (Endabschnitt E)
- 18e: Schaft (z.B. Nadelschaft)

- 20: zweites Mittel (zur Mengenerfassung des Zusatzstoffes Z)
- 20a: Aufhängung

- 22: drittes Mittel (zur zwangsweisen Förderung des Zusatzstoffes; z.B. Schlauchradpumpe)

- 24: viertes Mittel (zur Bereitstellung von sterilem Dampf D)
- 24a: fünftes Mittel (zur Bereitstellung von sterilem Kondensat K; z.B. Wärmeaustauscher)
- 24b: sechstes Mittel (zur Bereitstellung von Sterilluft)
- 24c: erste Leitung
- 24d: zweite Leitung
- 24e: dritte Leitung

- 26: erstes Absperrventil
- 28: zweites Absperrventil
- 30: drittes Absperrventil
- 32: viertes Absperrventil
- 34: Gully
- 36: Drehzahlregeleinrichtung
- 38: Temperaturfühler
- 40: erste Signalleitung
- 42: zweite Signalleitung
- 44: dritte Signalleitung
- 46: vierte Signalleitung
- 48: Steuerleitung
- 50: Steuereinrichtung

- 100: Dosierventil

- 102: Ventilgehäuse
- 102a: Rohrstutzen

- 104: Gehäuseverschlusskörper
- 104a: Zulaufkanal
- 104b: Ablaufkanal
- 104c: Laternenbohrung
- 104d: erste Sitzfläche
- 104e: zweite Sitzfläche
- 104f: Antriebskolben
- 104g: Durchtrittsöffnung

- 106: Antriebsgehäuse
- 106a: Klemmgehäuse

- 108: Überwurfmutter

- 110: Stempel
- 110a: Stempelausnehmung
- 110b: Stempelsitzfläche

- 112: Klemmringverbindung

- 114: erste Hülse
- 114a: Lagerzapfen
- 114b: Federteller
- 114c: Kanal

- 116: zweite Hülse
- 118: Andrückhülse

- 120: Verbindungsteil
- 120a: Zange

- 122: Scheibe
- 124: erster Sicherungsring
- 126: Antriebsfeder
- 128: Verschlusskolben
- 130: Gehäusedichtung
- 132: erste Kolbendichtung
- 134: zweite Kolbendichtung
- 136: erste Hülsendichtung
- 138: zweite Hülsendichtung
- 140: erstes Dichtungsmittel (für Endabschnitt E)
- 142: zweites Dichtungsmittel (für Endabschnitt E)
- 144: zweiter Sicherungsring
- 146: Antriebsraum
- 148: dritter Sicherungsring

- 200: Feder-Kolben-Antrieb
- 210: Klemm- und Anpresseinrichtung
- 220: erste Abdichteinrichtung
- 230: zweite Abdichteinrichtung

- D: steriler Dampf (Sterildampf)

- E: Endabschnitt (der Entnahmeleitung 18a)
- E1: endseitiger Teil des Endabschnittes E

- F: Fluid (L, D; K)

- I: Dosierstelle, allgemein
- I.1: erste Dosierstelle
- I.2: zweite Dosierstelle

- K: steriles Kondensat (Sterilkondensat)
- L: sterile Luft (Sterilluft)
- P: Basisprodukt

- R: anfänglicher Raum
- R1: Teilraum (vom anfänglichen Raum R abteilbar)
- RP: Produktraum

- SL: Steuerluft
- W: Weichwasser
- Z: Zusatzstoff

- H: erste Hubbewegung (Stempelhub)
- h: zweite Hubbewegung (Hub des Feder-Kolben-Antriebs 200)

## Patentansprüche

1. Verfahren zur aseptischen Dosierung eines flüssigen Zusatzstoffes (Z) in eine erzwungene Strömung eines Basisprodukts (P), wobei der Zusatzstoff (Z) unter aseptischen Bedingungen mittels einer Entnahmeleitung (18a) aus einem Bevorratungsbehälter (18) entnommen und über einen Endabschnitt (E) der Entnahmeleitung (18a) dem Basisprodukt (P), das einen Produktraum (RP) durchströmt, zugeführt wird,
**gekennzeichnet durch** die folgenden Behandlungsschritte für den Endabschnitt (E), bevor letzterer den Zusatzstoff (Z) dem Basisprodukt (P) zuführt:
(a) der Endabschnitt (E) wird von einer Seite ein Stück weit in einen anfänglichen Raum (R) temporär eingeführt oder dort permanent positioniert und an der Durchdringungsstelle zu diesem anfänglichen Raum (R) abgedichtet, wobei letzterer wahlweise zum Produktraum (RP) hin zu öffnen ist;
(b) ein in den anfänglichen Raum (R) abgedichtet hineinragender endseitiger Teil (E1) des Endabschnitts (E) wird zumindest sterilisiert, wobei alle Bereiche des anfänglichen Raumes (R) gleichfalls in diese Behandlung einbezogen sind;
(c) der endseitige Teil (E1) des Endabschnitts (E) wird in einen von dem anfänglichen Raum (R) wahlweise dicht abteilbaren Teilraum (R1) um ein Stück hineinverschoben, wobei der endseitige Teil (E1) am Ende der Verschiebung an der Durchdringungsstelle zum Teilraum (R1) mittels eines nach Behandlungsschritt (b) behandelten Dichtungsmittels abgedichtet ist;
(d) der Teilraum (R1) wird zum Produktraum (RP) hin geöffnet.

2. Verfahren nach Anspruch 1,
**dadurch gekennzeichnet,**
**dass** sich der Sterilisation nach Behandlungsschritt (b) eine Kühlung unter sterilen Bedingungen anschließt.

3. Verfahren nach Anspruch 2,
**dadurch gekennzeichnet,**
**dass** die Sterilisation mit sterilem Dampf (D) und die Kühlung mit Sterilluft (L) erfolgen.

4. Verfahren nach Anspruch 2 oder 3,
**dadurch gekennzeichnet,**
**dass** die Sterilisation und die Kühlung jeweils unterstromig des zu behandelnden endseitigen Teils (E1) temperaturüberwacht erfolgen.

5. Verfahren nach einem der Ansprüche 2 bis 4,
**dadurch gekennzeichnet,**
**dass** die Sterilisation bei einer Temperatur oberhalb von 125 °C durchgeführt wird und die Kühlung auf eine Temperatur unterhalb 50 °C erfolgt.

6. Verfahren nach einem der vorhergehenden Ansprüche,
**dadurch gekennzeichnet,**
**dass** die Entnahmeleitung (18a) bis zum Vollzug des Behandlungsschrittes (b) oder (c) frei bleibt vom Zusatzstoff (Z), und dass anschließend der Zusatzstoff (Z) bis zur Austrittsstelle des endseitigen Teils (E1) verbracht wird.

7. Verfahren nach Anspruch 6,
**dadurch gekennzeichnet,**
**dass** wahlweise eine Spülung des anfänglichen Raumes (R) mit sterilem Kondensat (K) unterhalb einer Temperatur von 50 °C durchgeführt wird.

8. Verfahren nach Anspruch 7,
**dadurch gekennzeichnet,**
**dass** im Anschluss an die Spülung des anfänglichen Raumes (R) dieser mittels Sterilluft (L) ausgeblasen wird.

9. Verfahren nach einem der vorhergehenden Ansprüche,
**dadurch gekennzeichnet,**
**dass** der Transport des Zusatzstoffes (Z) in der Entnahmeleitung (18a) zwangsweise erfolgt.

10. Verfahren nach einem der vorhergehenden Ansprüche,
**dadurch gekennzeichnet,**
**dass** der Zusatzstoff (Z) mengenproportional zum strömenden Basisprodukt (P) dosiert wird.

11. Dosiereinrichtung (10; 10.1, 10.2) zur Durchführung des Verfahrens nach einem der vorhergehenden Ansprüche, mit einem Produktleitungsabschnitt (12), der von dem Basisprodukt (P) zwangsweise durchströmt ist, mit wenigstens einem Bevorratungsbehälter (18; 18.1, 18.2) für den Zusatzstoff (Z), der jeweils über die Entnahmeleitung (18a) mit einer zugeordneten Dosierstelle (I; I.1, I.2), die an bzw. in dem Produktleitungsabschnitt (12) angeordnet ist, verbunden ist, mit dritten Mitteln (22) zur zwangsweisen Förderung des Zusatzstoffes (Z), mit ersten und zweiten Mitteln (14, 20) zur mengenmäßigen Erfassung des Basisprodukts (P) und des Zusatzstoffes (Z), mit vierten Mitteln (24) zur Bereitstellung von sterilem Dampf (D) und mit einer der Dosiereinrichtung (10; 10.1, 10.2) zugeordneten Steuereinrichtung (50),
**dadurch gekennzeichnet,**
**dass** die Dosierstelle (I; I.1, I.2) innerhalb eines Dosierventils (100) vorgesehen ist, das den Produktraum (PR), in den der Produktleitungsabschnitt (12) ein- und aus dem letzterer ausmündet, den anfänglichen Raum (R) und den Teilraum (R1) sowie eine Aufnahme des Endabschnitts (E) und des endseitigen Teils (E1 ausbildet.

12. Dosiereinrichtung nach Anspruch 11,
**dadurch gekennzeichnet,**
**dass** jener Teil des anfänglichen Raumes (R), der nach Abteilung des Teilraumes (R1) verbleibt, einen Zulauf- und einen Ablaufkanal (104a, 104b) aufweist, die wahlweise jeweils über ein zugeordnetes Absperrventil (26, 28) absperrbar sind und wobei der Zulaufkanal (104a) wenigstens mit dem vierten Mittel (24) zur Bereitstellung von sterilem Dampf (D) verbindbar ist.

13. Dosiereinrichtung nach Anspruch 12,
**dadurch gekennzeichnet,**
**dass** der Zulaufkanal (104a) wahlweise mit einem fünften Mittel (24a) zur Bereitstellung von sterilem Kondensat (K) und/oder einem sechsten Mittel (24b) zur Bereitstellung von Sterilluft (L) verbindbar ist.

14. Dosiereinrichtung nach Anspruch 12 oder 13,
**dadurch gekennzeichnet,**
**dass** im Ablaufkanal (104b) oder unterstromig unmittelbar im Anschluss an den Ablaufkanal (104b) ein Temperaturfühler (38) angeordnet ist.

15. Dosiereinrichtung nach einem der Ansprüche 11 bis 14,
**dadurch gekennzeichnet,**
**dass** der Bevorratungsbehälter (18) und wenigstens die Entnahmeleitung (18a) im Anlieferungszustand eine sterile Einheit bilden.

16. Dosiereinrichtung nach einem der Ansprüche 11 bis 15,
**dadurch gekennzeichnet,**
**dass** der Endabschnitt (E) mit seinem integralen endseitigen Teil (E1) einstückig als Injektionsnadel (18b) oder Rohr mit glattem Ende (18c) ausgebildet ist.

17. Dosiereinrichtung nach einem der Ansprüche 11 bis 15,
**dadurch gekennzeichnet,**
**dass** der endseitige Teil (E1) separat vom Endabschnitt (E) und mit einer steuerbaren Auslassöffnung ausgeführt ist, dass der Endabschnitt (E) als Injektionsnadel (18b) oder Rohr mit glattem Ende (18c) oder als Adapter (18d) zur Herstellung einer fluidgängigen Verbindung mit dem endseitigen Teil (E1) ausgebildet ist, und dass der Endabschnitt (E) mit dem endseitigen Teil (E1) fluidgängig verbunden ist.

18. Dosiereinrichtung nach einem der Ansprüche 11 bis 17,
**dadurch gekennzeichnet,**
**dass** der Bevorratungsbehälter (18) in Form eines flexiblen Beutels ausgebildet ist, und dass der mit dem Zusatzstoff (Z) gefüllte Beutel (18) und wenigstens die Entnahmeleitung (18a) in einer PE-Umverpackung dicht eingeschlossen und zum Zwecke der äußerlichen Sterilisierung einer Gammabestrahlung unterzogen sind.

19. Dosierventil (100) für eine Dosiereinrichtung (10) nach einem der Ansprüche 11 bis 18,
**gekennzeichnet**
• **durch** ein in dem Produktleitungsabschnitt (12) angeordnetes, den Produktraum (RP) berandendes Ventilgehäuse (102),
• **durch** einen das Ventilgehäuse (102) einerseits verschließenden Gehäuseverschlusskörper (104) mit einer Durchtrittsöffnung (104g) zum Produktraum (RP),
• **durch** eine erste Hülse (114), die koaxial zur Durchtrittsöffnung (104g) angeordnet ist und verschieblich und abgedichtet von außen in den Gehäuseverschlusskörper (104) eingreift und mittels eines druckmittelbeaufschlagten Feder-Kolben-Antriebs (200) angetrieben ist und damit eine zweite Hubbewegung (h) ausführt,
• **durch** eine Klemm- und Anpresseinrichtung (210), die vom Endabschnitt (E) durchdrungen ist und letzteren radial und axial festlegt,
• **durch** eine innen in der ersten Hülse (114) Aufnahme findende erste Abdichteinrichtung (220), die vom Endabschnitt (E) durchdrungen ist und die im Zusammenwirken mit der Klemm- und Anpresseinrichtung (210) den endseitigen Teil (E1) des Endabschnittes (E) gegenüber der ersten Hülse (114) unmittelbar oder mittelbar durch ein axial und radial verpresstes erstes Dichtungsmittel (140) abdichtet,
• **durch** einen Stempel (110), der von der der Durchtrittsöffnung (104g) abgewandten Seite des Ventilgehäuses (102) in dieses eingreift und nach einer ersten Hubbewegung (H) in seiner einen Endlage im Umfangsbereich der Durchtrittsöffnung (104g), der als erste Sitzfläche (104d) ausgebildet ist, mit einer Stempelsitzfläche (110b) anliegt,
• **durch** eine in der der Durchtrittsöffnung (104g) zugewandten Stirnfläche des Stempels (110) vorgesehene raumbildende Stempelausnehmung (110a), die zusammen mit einem in dem Gehäuseverschlusskörper (104) zwischen der Durchtrittsöffnung (104g) und dem stirnseitigen, verschieblichen Ende der ersten Hülse (114) befindlichen Raum den anfänglichen Raum (R) ausbildet, wobei der im Gehäuseverschlusskörper (104) befindliche Raum über einen Zulauf- und den Ablaufkanal (104a, 104b) jeweils mit der Umgebung des Dosierventils (100) verbunden ist,
• **durch** einen Verschlusskolben (128), der von dem endseitigen Teil (E1) des Endabschnittes (E) durchdrungen und auf einem an der ersten Hülse (114) endseitig angeordneten, in den anfänglichen Raum (R) hineinragenden Lagerzapfen (114a) angeordnet ist und in seiner durch eine Antriebsfeder (126) im Feder-Kolben-Antrieb (200) nach der zweiten Hubbewegung (h) erzeugten Endlage, der Schließstellung, im Umfangsbereich der Durchtrittsöffnung (104g), der als zweite Sitzfläche (104e) ausgebildet ist, dichtend anliegt, wobei der Verschlusskolben (128) in seiner Schließstellung zwischen sich und der Stempelausnehmung (110a) den Teilraum (R1) von dem anfänglichen Raum (R) abteilt und der Verschlusskolben (128) gegenüber dem endseitigen Teil (E1) durch ein axial und radial verpresstes zweites Dichtungsmittel (142) im Rahmen einer zweiten Abdichteinrichtung (230) abgedichtet ist und
• **durch** eine mit der zweiten Hubbewegung (h) erzeugte andere Endlage des Verschlusskolbens (128), der Offenstellung, in der sich das zweite Dichtungsmittel (142) spaltweit von dem endseitigen Teil (E1) gelöst hat und alle Oberflächen und Bereiche des anfänglichen Raumes (R) der Beaufschlagung mit einem Fluid (F), bestehend aus sterilem Dampf (D) oder Sterilluft (L) oder sterilem Kondensat (K), zugänglich sind.

20. Dosierventil nach Anspruch 19,
**dadurch gekennzeichnet,**
**dass** der dem Ventilgehäuse (102) abgewandte Endabschnitt des Gehäuseverschlusskörpers (104) als Antriebskolben (104f) des Feder-Kolben-Antriebs (200) ausgebildet und von der ersten Hülse (114) verschieblich und abgedichtet durchdrungen ist, dass ein zugeordnetes Antriebsgehäuse (106) topfförmig ausgebildet und von der ersten Hülse (114) durchdrungen und mit dieser form- und kraftschlüssig verbunden ist, und dass sich die Antriebsfeder (126) einerseits am Antriebskolben (104f) und andererseits an der ersten Hülse (114) in der Weise abstützt, dass die Rückstellkraft der Antriebsfeder (126) den Verschlusskolben (128) in seine Schließstellung überführt.

21. Dosierventil nach Anspruch 20,
**dadurch gekennzeichnet,**
**dass** das Antriebsgehäuse (106) auf seiner dem Antriebskolben (104f) abgewandten Seite in ein topfförmiges, durch eine Überwurfmutter (108) begrenztes Klemmgehäuse (106a) der Klemm- und Anpresseinrichtung (210) übergeht, und dass die Überwurfmutter (108) über konische Wirkflächen auf eine von dem Endabschnitt (E) durchdrungene Zange (120a) dergestalt einwirkt, dass der Endabschnitt (E) durch die Zange (120a) axial und radial festgelegt ist.

22. Dosierventil nach Anspruch 21,
**dadurch gekennzeichnet,**
**dass** die Zange (120a) mit dem einen Ende einer in der ersten Hülse (114) aufgenommenen, von dem Endabschnitt (E) vollständig durchdrungenen zweiten Hülse (116) verbunden ist und axial derart auf letztere einwirkt, dass diese mit ihrem anderen Ende das erste Dichtungsmittel (140) axial und radial verpresst.

23. Dosierventil nach einem der Ansprüche 19 bis 22,
**dadurch gekennzeichnet,**
**dass** der Verschlusskolben (128) und der in diesen eingreifende Lagerzapfen (114a) einen den endseitigen Teil (E1) umschließenden keilförmigen Ringraum bilden, der sich von innen nach außen verjüngt und das zweite Dichtungsmittel (142) aufnimmt, dass in der Schließstellung des Verschlusskolbens (128) die Flanken des keilförmigen Ringraumes so weit angenähert sind, dass das zweite Dichtungsmittel (142) axial und radial verpresst und damit gegen den endseitigen Teil (E1) abgedichtet ist, und dass in der Offenstellung des Verschlusskolbens (128) die Flanken des keilförmigen Ringraumes so weit voneinander entfernt sind, dass sich das zweite Dichtungsmittel (142) aufweitet und spaltweit von dem endseitigen Teil (E1) entfernt ist.

## Claims

1. A method for the aseptic measured delivery of a liquid additive (Z) into a forced flow of a base product (P), wherein the additive (Z) is removed from a storage container (18) under aseptic conditions by means of a removal conduit (18a) and is fed via an end section (E) of the removal conduit (18a) to the base product (P), which flows through a product chamber (RP),
**characterized by** the following treatment steps for the end section (E), before the latter feeds the additive (Z) to the base product (P):
**(a)** the end section (E) is temporarily introduced a certain distance into an initial chamber (R) from one side or is permanently positioned therein and is sealed at the penetration point to this initial chamber (R), wherein the latter can optionally be opened towards the product chamber (RP);
**(b)** a terminal part (E1) of the end section (E) that projects into the initial chamber (R) in a sealed manner is at least sterilized, wherein all regions of the initial chamber (R) are also included in this treatment;
**(c)** the terminal part (E1) of the end section (E) is slid a certain distance into a partial chamber (R1) that can optionally be sealably divided from the initial chamber (R), the terminal part (E1) being sealed at the end of the sliding action at the penetration point to the partial chamber (R1) by means of a sealing means treated as per treatment step (b);
**(d)** the partial chamber (R1) is opened towards the product chamber (RP).

2. The method according to claim 1,
**characterized in that**
a cooling under sterile conditions follows the sterilization according to treatment step (b).

3. The method according to claim 2,
**characterized in that**
the sterilization takes place with sterile steam (D) and the cooling with sterile air (L).

4. The method according to claim 2 or 3,
**characterized in that**
the sterilization and the cooling take place in a temperature-monitored manner respectively downstream of the terminal part (E1) to be treated.

5. The method according to one of claims 2 to 4,
**characterized in that**
the sterilization is performed at a temperature above 125° C and the cooling takes place to a temperature below 50° C.

6. The method according to one of the previous claims,
**characterized in that**
the removal conduit (18a) remains free of the additive (Z) up to completion of the treatment step (b) or (c) and that the additive (Z) is then applied up to the discharge point of the terminal part (E1).

7. The method according to claim 6,
**characterized in that**
a flushing of the initial chamber (R) with sterile condensate (K) is optionally performed below a temperature of 50° C.

8. The method according to claim 7,
**characterized in that**
it is blown out by means of sterile air (L) after the flushing of the initial chamber (R).

9. The method according to one of the previous claims,
**characterized in that**
the transport of the additive (Z) takes place in a forced manner in the removal conduit (18a).

10. The method according to one of the previous claims,
**characterized in that**
the additive (Z) is delivered in a measured manner to the base product (P) flow-proportional to the quantity.

11. A metering device (10; 10.1, 10.2) for performing the method according to one of the previous claims, with a product conduit section (12), through which the base product (P) forcibly flows, with at least one storage container (18; 18.1, 18.2) for the additive (Z), which is connected with an associated metering point (I; I.1, I.2) respectively via the removal conduit (18a), which is arranged on or respectively in the product conduit section (12), with third means (22) for forcibly conveying the additive (Z), with first and second means (14, 20) for the quantity-based capturing of the base product (P) and of the additive (Z), with fourth means (24) for providing sterile steam (D) and with a control device (50) assigned to the dosing device (10; 10.1, 10.2),
**characterized in that**
the metering point (I; I.1, I.2) is provided within a metering valve (100), which forms the product chamber (PR), into which the product conduit section (12) opens and from which it exits, the initial chamber (R) and the partial chamber (R1) as well as the receiver of the end section (E) and of the terminal side (E1).

12. The metering device according to claim 11,
**characterized in that**
that part of the initial chamber (R) remaining after division of the partial chamber (R1), has an infeed and a discharge channel (104a, 104b), which are optionally blockable via an associated stop valve (26, 28) and wherein the infeed channel (104a) can be connected at least with the fourth means (24) for providing sterile steam (D).

13. The metering device according to claim 12,
**characterized in that**
the infeed channel (104a) can be optionally connected with a fifth means (24a) for providing sterile condensate (K) and/or a sixth means (24b) for providing sterile air (L).

14. The metering device according to claim 12 or 13,
**characterized in that**
a temperature sensor (38) is arranged immediately following the discharge channel (104b) in the discharge channel (104b) or downstream.

15. The metering device according to one of claims 11 to 14,
**characterized in that**
the storage container (18) and at least the removal conduit (18a) form a sterile unit in the delivery state.

16. The metering device according to one of claims 11 to 15,
**characterized in that**
the end section (E) with its integral terminal part (E1) is designed as one piece as an injection needle (18b) or a tube with a smooth end (18c).

17. The metering device according to one of claims 11 to 15,
**characterized in that**
the terminal part (E1) is designed separately from the end section (E) and with a controllable discharge opening, that the end section (E) is designed as an injection needle (18b) or a tube with a smooth end (18c) or as an adapter (18d) for establishing a fluid-accessible connection with the terminal part (E1) and that the end section (E) is connected in a fluid-accessible manner with the terminal part (E1).

18. The metering device according to one of claims 11 to 17,
**characterized in that**
the storage container (18) is designed in the form of a flexible bag and that the bag (18) filled with the additive (Z) and at least the removal conduit (18a) are sealed in a PE overwrap and subjected to gamma irradiation for the purpose of external sterilization.

19. A metering valve (100) for a metering device (10) according to one of claims 11 to 18, **characterized by**
• a valve housing (102) arranged in the product conduit section (12) and bordering the product chamber (RP),
• a housing closure body (104) closing the valve housing (102) on one side with a passage opening (104g) to the product chamber (RP),
• a first sleeve (114), which is arranged coaxially to the passage opening (104g) and engages in the housing closure body (104) in a displaceable and sealed manner from outside and is driven by means of a spring piston drive (200) supplied with pressurizing agent and thus executes a second lifting movement (h),
• a clamping and pressing device (210), which is penetrated by the end section (E) and fixes the latter radially and axially,
• a first sealing device (220) being received inside the first sleeve (114), which is penetrated by the end section (E) and which together with the clamping and pressing device (210) seals the terminal part (E1) of the end section (E) with respect to the first sleeve (114) directly or indirectly through an axially and radially pressed first sealing means (140),
• a spool (110), which engages in it from the side of the valve housing (102) facing away from the passage opening (104g), and is in abutment with a spool seating (110b) after a first lifting movement (H) in its one end position in the circumferential area of the passage opening (104g), which is designed as a first seating (104d),
• a chamber-forming spool recess (110a) provided in the front surface of the spool (110) facing the passage opening (104g), which forms the initial chamber (R) together with a chamber located in the housing closure body (104) between the passage opening (104g) and the front-side, displaceable end of the first sleeve (114), wherein the chamber located in the housing closure body (104) is connected respectively with the surrounding area of the metering valve (100) via an infeed and a discharge channel (104a, 104b),
• a closure piston (128), which is penetrated by the terminal part (E1) of the end section (E) and is arranged on a bearing journal (114a) arranged on the end side on the first sleeve (114) and protruding into the initial chamber (R) and is in sealing abutment in its end position, the closed position, generated by a drive spring (126) in the spring piston drive (200) after the second lifting movement (h), in the circumferential area of the passage opening (104g), which is designed as the second seating (104e), wherein the closure piston (128) in its closed position between itself and the spool recess (110a) divides the partial chamber (R1) from the initial chamber (R) and the closure piston (128) is sealed off with respect to the terminal part (E1) by an axially and radially pressed second sealing means (142) within the framework of a second sealing device (230) and
• another end position of the closure piston (128), the open position, generated with the second lifting movement (h), in which the second sealing means (142) is released from the terminal part (E1) by a gap's width and all surfaces and areas of the initial chamber (R) are accessible for application of a fluid (F), consisting of sterile steam (D) or sterile air (L) or sterile condensate (K).

20. The metering valve according to claim 19,
**characterized in that**
the end section of the housing closure body (104) facing away from the valve housing (102) is designed as a drive piston (104f) of the spring piston drive (200) and is penetrated in a shiftable and sealed manner by the first sleeve (114), that an assigned drive housing (106) is designed in a pot-like manner and penetrated by the first sleeve (114) and connected with it in a form- and force-fitting manner, and that the drive spring (126) on one side on the drive piston (104f) and on the other side on the first sleeve (114) is supported in a manner such that the reset force of the drive spring (126) transfers the closure piston (128) into its closed position.

21. The metering valve according to claim 20,
**characterized in that**
the drive housing (106) on its side facing away from the drive piston (104f) transitions into a pot-like clamp housing (106a) of the clamping and pressing device (210) delimited by a cap nut (108) and that the cap nut (108) acts via conical effective areas on a collet (120a) penetrated by the end section (E) such that the end section (E) is determined axially and radially by the collet (120a).

22. The metering valve according to claim 21,
**characterized in that**
the collet (120a) is connected with the one end of a second sleeve (116) received in the first sleeve (114) and completely penetrated by the end section (E) and acts axially on the latter such that it presses the first sealing means (140) axially and radially with its other end.

23. The metering valve according to one of claims 19 to 22,
**characterized in that**
the closure piston (128) and the bearing journal (114a) engaging in it form a wedge-shaped annular space surrounding the terminal part (E1), which tapers from inside to outside and receives the second sealing means (142), that in the closed position of the closure piston (128) the flanks of the wedge-shaped annular space converge far enough that the second sealing means (142) is pressed axially and radially and is thus sealed against the terminal part (E1) and that in the open position of the closure piston (128) the flanks of the wedge-shaped annular space are spaced apart from each other far enough that the second sealing means (142) broadens and is spaced apart from the terminal part (E1) by a gap's width.

## Revendications

1. Procédé pour le dosage aseptique d'un additif liquide (Z) dans un écoulement forcé d'un produit de base (P), l'additif (Z) étant prélevé à partir d'un récipient de stockage (18) dans des conditions aseptiques au moyen d'une conduite de prélèvement (18a) et étant ajouté au produit de base (P) s'écoulant à travers une chambre de produit (RP), par le biais d'une section d'extrémité (E) de la conduite de prélèvement (18a),
**caractérisé par** les étapes de traitement suivantes pour la section d'extrémité (E), avant que celle-ci ne délivre l'additif (Z) au produit de base (P) :
(a) la section d'extrémité (E) est temporairement insérée par un côté partiellement dans une chambre initiale (R), où elle est ensuite positionnée de façon permanente et étanchéifiée au niveau du point de passage vers cette chambre initiale (R), celle-ci pouvant être ouverte sélectivement vers la chambre de produit (RP) ;
(b) une partie côté extrémité (E1) de la section d'extrémité (E) faisant saillie dans la chambre initiale (R) de façon étanche est au moins stérilisée, les régions de la chambre initiale (R) étant également incluses en totalité dans ce traitement ;
(c) la partie côté extrémité (E1) de la section d'extrémité (E) est insérée partiellement dans une chambre partielle (R1) séparable de façon étanche par rapport à la chambre initiale (R), la partie côté extrémité (E1) étant étanchéifiée au niveau du point de passage vers la chambre partielle (R1) à la fin de l'insertion, à l'aide d'un moyen d'étanchéité traité selon l'étape de traitement (b) ;
(d) la chambre partielle (R1) est ouverte sur la chambre de produit (RP).

2. Procédé selon la revendication 1,
**caractérisé en ce que**
la stérilisation selon l'étape de traitement (b) est suivie d'un refroidissement dans des conditions stériles.

3. Procédé selon la revendication 2,
**caractérisé en ce que**
la stérilisation est réalisée avec une vapeur stérile (D) et le refroidissement avec de l'air stérile (L).

4. Procédé selon la revendication 2 ou 3,
**caractérisé en ce que**
la stérilisation et le refroidissement sont réalisés en aval de la partie côté extrémité (E1) à traiter, en surveillant la température.

5. Procédé selon l'une des revendications 2 à 4,
**caractérisé en ce que**
la stérilisation est réalisée à une température supérieure à 125°C, et le refroidissement à une température inférieure à 50°C.

6. Procédé selon l'une des revendications précédentes,
**caractérisé en ce que**
la conduite de prélèvement (18a) reste exempte d'additif (Z) jusqu'à la fin de l'étape de traitement (b) ou (c), et **en ce que** l'additif (Z) est ensuite guidé jusqu'au point de sortie de la partie côté extrémité (E1).

7. Procédé selon la revendication 6,
**caractérisé en ce que**
un rinçage de la chambre initiale (R) avec un condensat stérile (K) à une température inférieure à 50°C peut être réalisé au choix.

8. Procédé selon la revendication 7,
**caractérisé en ce que**
suite au rinçage de la chambre initiale (R), celle-ci est purgée avec de l'air stérile (L).

9. Procédé selon l'une des revendications précédentes,
**caractérisé en ce que**
le transport de l'additif (Z) dans la conduite de prélèvement (18a) est effectué de manière forcée.

10. Procédé selon l'une des revendications précédentes,
**caractérisé en ce que**
l'additif (Z) est dosé en quantités proportionnelles au produit de base (P) en écoulement.

11. Dispositif de dosage (10 ; 10.1 ; 10.2) pour l'exécution du procédé selon l'une des revendications précédentes, comprenant une section de conduite de produit (12) traversée de manière forcée par le produit de base (P), au moins un récipient de stockage (18 ; 18.1 ; 18.2) pour l'additif (Z), lequel est respectivement relié à un point de dosage (I ; 1.1 ; 1.2) correspondant situé sur ou dans la section de conduite de produit (12) par le biais de la conduite de prélèvement (18a), des troisièmes moyens (22) pour l'écoulement forcé de l'additif (Z), des premiers et deuxièmes moyens (14, 20) pour la détection quantitative du produit de base (P) et de l'additif (Z), des quatrièmes moyens (24) pour l'apport de vapeur stérile (D) et un dispositif de commande (50) attribué au dispositif de dosage (10 ; 10.1, 10.2),
**caractérisé en ce que**
le point de dosage (I ; I.1 ; 1.2) est prévu à l'intérieur d'une soupape de dosage (100) formant la chambre de produit (PR) dans laquelle la section de conduite de produit (12) débouche et d'où celle-ci repart, la chambre initiale (R) et la chambre partielle (R1) ainsi qu'une admission de la section d'extrémité (E) et de la partie côté extrémité (E1).

12. Dispositif de dosage selon la revendication 11,
**caractérisé en ce que**
la partie de la chambre initiale (R) restant suite à la séparation de la chambre partielle (R1) comporte un canal d'alimentation et un canal d'évacuation (104a, 104b), lesquels peuvent être verrouillés respectivement de manière sélective au moyen d'une soupape d'arrêt (26, 28) correspondante, le canal d'alimentation (104a) pouvant être relié au moins au quatrième moyen (24) pour l'apport de vapeur stérile (D).

13. Dispositif de dosage selon la revendication 12,
**caractérisé en ce que**
le canal d'alimentation (104a) peut être relié de manière sélective à un cinquième moyen (24a) pour l'apport de condensat stérile (K) et/ou à un sixième moyen (24b) pour l'apport d'air stérile (L).

14. Dispositif de dosage selon la revendication 12 ou 13,
**caractérisé en ce que**
une sonde de température (38) est disposée dans le canal d'évacuation (104b) ou en aval directement à la suite du canal d'évacuation (104b).

15. Dispositif de dosage selon l'une des revendications 11 à 14,
**caractérisé en ce que**
le récipient de stockage (18) et au moins la conduite de prélèvement (18a) forment une unité stérile dans l'état de livraison.

16. Dispositif de dosage selon l'une des revendications 11 à 15,
**caractérisé en ce que**
la section d'extrémité (E) avec la partie côté extrémité (E1) intégrale est conçue d'un seul tenant comme une aiguille d'injection (18b) ou comme un tube à extrémité lisse (18c).

17. Dispositif de dosage selon l'une des revendications 11 à 15,
**caractérisé en ce que**
la partie côté extrémité (E1) est conçue séparément de la section d'extrémité (E) et avec une ouverture d'évacuation contrôlable, **en ce que** la section d'extrémité (E) est conçue comme une aiguille d'injection (18b) ou comme un tube à extrémité lisse (18c) ou comme un adaptateur (18d) destiné à établir une liaison fluidique avec la partie côté extrémité (E1), et **en ce que** la section d'extrémité (E) est reliée fluidiquement à la partie côté extrémité (E1).

18. Dispositif de dosage selon l'une des revendications 11 à 17,
**caractérisé en ce que**
le récipient de stockage (18) est conçu sous la forme d'une poche souple, et **en ce que** la poche (18) remplie d'additif (Z) et au moins la conduite de prélèvement (18a) sont enfermées de façon étanche dans un suremballage en PE et soumises à un rayonnement gamma pour une stérilisation extérieure.

19. Soupape de dosage (100) pour un dispositif de dosage (10) selon l'une des revendications 11 à 18,
**caractérisée par**
• un boîtier de soupape (102) bordant la chambre de produit (RP), disposé dans la section de conduite de produit (12),
• un corps de fermeture de boîtier (104) fermant le boîtier de soupape (102) sur un côté, avec une ouverture de passage (104g) vers la chambre de produit (RP),
• une première douille (114) disposée coaxialement à l'ouverture de passage (104g) et s'engageant de façon coulissante et étanche depuis l'extérieur dans le corps de fermeture de boîtier (104), laquelle est entraînée au moyen d'un entraînement à ressort et piston (200) soumis à un fluide sous pression, exécutant ainsi un deuxième mouvement de course (h),
• un dispositif de serrage et de pression (210) traversé par la section d'extrémité (E) et fixant celle-ci radialement et axialement,
• un premier dispositif d'étanchéité (220) reçu à l'intérieur de la première douille (114), lequel est traversé par la section d'extrémité (E) et assure l'étanchéité de la partie côté extrémité (E1) de la section d'extrémité (E) par rapport à la première douille (114), directement ou indirectement par le biais d'un premier moyen d'étanchéité (140) comprimé axialement et radialement, en coopération avec le dispositif de serrage et de pression (210),
• un tampon (110) s'engageant dans le boîtier de soupape (102) par le côté de celui-ci qui est opposé à l'ouverture de passage (104g), et s'appliquant dans sa position finale avec une surface d'appui de tampon (110b) dans la région périphérique de l'ouverture de passage (104g) formée comme une première surface d'appui (104d), après un premier mouvement de course (H),
• un évidement de tampon (110a) formant un espace, prévu dans la surface frontale du tampon (110) qui est tournée vers l'ouverture de passage (104g), lequel forme la chambre initiale (R) ensemble avec une chambre située dans le corps de fermeture de boîtier (104) entre l'ouverture de passage (104g) et l'extrémité avant déplaçable de la première douille (114), l'espace situé dans le corps de fermeture de boîtier (104) étant relié respectivement à l'environnement de la soupape de dosage (100) par le biais d'un canal d'alimentation et du canal d'évacuation (104a, 104b),
• un piston de fermeture (128) traversé par la partie côté extrémité (E1) de la section d'extrémité (E) et disposé sur un tourillon (114a) disposé côté extrémité sur la première douille (114) et faisant saillie dans la chambre initiale (R), lequel s'applique de façon étanche dans la région périphérique de l'ouverture de passage (104g) formant la deuxième surface d'appui (104e), dans sa position finale, la position de fermeture, atteinte après le deuxième mouvement de course à l'aide d'un ressort d'entraînement (126) dans l'entraînement à ressort et piston (200), le piston de fermeture (128) séparant la chambre partielle (R1) d'avec la chambre initiale (R) dans sa position de fermeture entre lui-même et l'évidement de tampon (110a), et le piston de fermeture (128) étant étanchéifié par rapport à la partie côté extrémité (E1) par un deuxième moyen d'étanchéité (142) dans le cadre d'un deuxième dispositif d'étanchéité (230), et
• une autre position finale du piston de fermeture (128) atteinte avec le deuxième mouvement de course (h), la position d'ouverture, dans laquelle le deuxième moyen d'étanchéité (142) s'est détaché de la partie côté extrémité (E1) sur une largeur de fente, et toutes les surfaces et régions de la chambre initiale (R) sont accessibles pour être soumises à un fluide (F) composé de vapeur stérile (D) ou d'air stérile (L) ou de condensat stérile (K).

20. Soupape de dosage selon la revendication 19,
**caractérisée en ce que**
la section d'extrémité du corps de fermeture de boîtier (104) qui est opposée au boîtier de soupape (102) est formée comme un piston d'entraînement (104f) de l'entraînement à ressort et piston (200) et traversée de façon coulissante et étanche par la première douille (114), **en ce qu'**un boîtier d'entraînement (106) correspondant et conçu en forme de pot et traversé par la première douille (114) tout en étant relié à celle-ci par complémentarité de forme ou à force, et **en ce que** le ressort d'entraînement (126) s'appuie d'une part sur le piston d'entraînement (104f) et d'autre part sur la première douille (114) de telle façon que la force de rappel du ressort d'entraînement (126) fait passer le piston de fermeture (128) dans sa position de fermeture.

21. Soupape de dosage selon la revendication 20,
**caractérisée en ce que** le boîtier d'entraînement (106), sur son côté opposé au piston d'entraînement (104f), se prolonge en un boîtier de serrage (106a) en forme de pot du dispositif de serrage et de pression (210) délimité par un écrou-raccord (108), et **en ce que** l'écrou-raccord (108) agit avec des surfaces actives coniques sur une pince (120a) traversée par la section d'extrémité (E) de telle façon que la section d'extrémité (E) est fixée axialement et radialement par la pince (120a).

22. Soupape de dosage selon la revendication 21,
**caractérisée en ce que**
la pince (120a) est reliée à une extrémité d'une deuxième douille (116) reçue dans la première douille (114) et entièrement traversée par la section d'extrémité (E), et agit axialement sur celle-ci de manière à ce que celle-ci comprime le premier moyen d'étanchéité (140) axialement et radialement avec son autre extrémité.

23. Soupape de dosage selon l'une des revendications 19 à 22,
**caractérisée en ce que**
le piston de fermeture (128) et le tourillon (114a) s'engageant dans celui-ci forment un espace annulaire cunéiforme entourant la partie côté extrémité (E1), lequel s'affine de l'intérieur vers l'extérieur et reçoit le deuxième moyen d'étanchéité (142), **en ce que** dans la position de fermeture du piston de fermeture (128), les flancs de l'espace annulaire cunéiforme sont rapprochés l'un de l'autre au point que le deuxième moyen d'étanchéité (142) est comprimé axialement et radialement et étanchéifié par rapport à la partie côté extrémité (E1), et **en ce que** dans la position d'ouverture du piston de fermeture (128), les flancs de l'espace annulaire cunéiforme sont éloignés l'un de l'autre au point que le deuxième moyen d'étanchéité (142) s'élargit et se retrouve à une distance de fente de la partie côté extrémité (E1).
